# EUROPEAN PATENT APPLICATION

(11) **EP 3 895 711 A1**
(43) Date of publication of application: **20.10.2021**
(21) Application number: 19896822.4
(22) Date of filing: 10.12.2019
(51) Int. Cl.: A61K 31/724, C12P 19/18, C12R 1/00, C12N 5/10, A23K 20/163, A23L 33/125, A61K 8/73, A61K 47/40, C12N 1/15, C12N 1/19, C12N 1/20, C12N 1/21, C12N 15/52, C08B 37/16, C12N 9/00

(54) **CYCLOISOMALTOTETRAOSE, CYCLOISOMALTOTETRAOSE PRODUCTION ENZYME, AND PRODUCTION METHODS AND USES THEREOF**

(30) Priority: 13.12.2018 JP 2018233145; 16.08.2019 JP 2019149422
(71) Applicant: Hayashibara Co., Ltd., Okayama-shi, Okayama 702-8006 (JP)
(72) Inventor: FUJITA Akihiro, Okayama-shi Okayama 702-8006 (JP); KAWASHIMA Akira, Okayama-shi Okayama 702-8006 (JP); FUJISAWA Hiroki, Okayama-shi Okayama 702-8006 (JP); MORI Tetsuya, Okayama-shi Okayama 702-8006 (JP); NISHIMOTO Tomoyuki, Okayama-shi Okayama 702-8006 (JP)
(74) Representative: Daniels, Jeffrey Nicholas
(86) International application number: PCT/JP2019/048218
(87) International publication number: WO 2020/122050

(57) **Abstract**

The present invention has objects to provide a novel cyclic saccharide composed of D-glucose as constituents, a novel enzyme forming the cyclic saccharide, processes for producing the same, and uses thereof. The present invention solves the above objects by providing cycloisomaltotetraose having a structure in which four D-glucose molecules are bound in cyclic form *via* α-1,6 glucosidic linkages, a cycloisomaltotetraose-forming enzyme, a DNA encoding the enzyme, processes for producing the same, and uses thereof.

## Description

### Technical Field

The present invention relates to a novel cyclic saccharide composed of D-glucose as constituents, a novel enzyme which forms the cyclic saccharide, their preparation and uses.

### Background Art

In the field of saccharides, various cyclic saccharides, produced by enzymatic reactions using α-1,4 glucan composed by D-glucose such as starch or partial starch hydrolysates as material, are known. Non-Patent Literature 1 discloses α- β- and γ-cyclodextrins, saccharides having cyclic structures that 6 to 8 D-glucose molecules are bound in cyclic form *via* α-1,4 glucosidic linkages, obtainable by allowing "macerans amylase" to act on starch. Currently, these cyclodextrins are produced on an industrial scale and utilized in various applications using their characteristics, such as non-reducing power, tastelessness, and inclusion ability. While, Patent Literature 1 discloses a cyclic tetrasaccharide, having a structure of binding four glucose molecules *via* alternating α-1,3 and α-1,6 glucosidic linkages, i.e., *cyclo*{→6)-α-D-glucopyranosyl-(1→3)-α-D-glucopyranosyl-(1→6)-α-D-glucopyranosyl-(1→3)-α-D-glucopyranosyl-(1→}, which is obtainable by allowing α-isomaltosylglucosaccharide-forming enzyme and α-isomaltosyl-transferring enzyme to act on starch or its partial hydrolysate. Further, Patent Literature 2 discloses a cyclic tetrasaccharide, having a structure of binding four glucose molecules *via* alternating α-1,4 and α-1,6 glucosidic linkages, i.e., *cyclo*{→6)-α-D-glucopyranosyl-(1→4)-α-D-glucopyranosyl-(1→6)-α-D-glucopyranosyl-(1→4)-α-D-glucopyranosyl-(1→} (also known as "cyclic maltosylmaltose"), which is obtainable by allowing a cyclic maltosylmaltose-forming enzyme to act on starch or its partial hydrolysates. Furthermore, Patent Literature 3 discloses a cyclic pentasaccharide having a structure of binding C-1 position of the reducing end glucose of maltopentaose to the hydroxy group at the C-6 position of the non-reducing end glucose in the same maltopentaose molecule *via* α-1,6 glucosidic linkage to form cyclic structure ("isocyclomaltopentaose") and a cyclic hexasaccharide having a structure of binding C-1 position of the reducing end glucose of maltohexaose to the hydroxy group at the C-6 position of the non-reducing end glucose in the same maltohexaose molecule *via* α-1,6 glucosidic linkage to form cyclic structure ("isocyclomaltohexaose"). These cyclic saccharides have an inclusion ability because they have a cyclic structure, and exhibit the effect of stabilizing the volatile organic substances. Further, since they are non-reducing saccharides, they can be used for processing without causing an aminocarbonyl reaction, browning, and degradation. Therefore, they are expected to be utilized in various fields by taking advantage of each characteristic.

On the other hand, as another α-glucan composed by D-glucose, dextran, an α-1,6 glucan having a basic structure in which D-glucose molecules are bound in a chain *via* α-1,6 glucosidic linkages, has been known. In addition, cycloisomaltooligosaccharides (also called as "cyclic isomaltooligosaccharide" or "cyclodextran") have been reported as cyclic saccharides obtainable by an enzymatic reaction using dextran as a material. Patent Literature 4 and Non-Patent Literature 2 disclose cycloisomaltoheptaose (CI-7), cycloisomaltooctaose (CI-8) and cycloisomaltononaose (CI-9), cyclic saccharides in which 7 to 9 D-glucose molecules were bound *via* α-1,6 glucosidic linkages, obtainable by allowing an enzyme derived from a microorganism of the genus *Bacillus.* Further, Patent Literature 5 and Non-Patent Literature 3 disclose CI-10 to CI-16, having higher glucose polymerization degrees of 10 to 16, and Patent Literature 6 discloses CI-13 to CI-33, having more higher glucose polymerization degrees of 13 to 33. However, cycloisomaltooligosaccharides having glucose polymerization degrees of less than 7 have not been known at all.

If other cyclic saccharide having D-glucose molecule as a component, distinct from the above saccharides, would be provided, application thereof for various uses can be expected.

### Prior Art Literature

### Patent Literature

- Patent Literature 1:: International Patent Application WO2001/90338A1 Pamphlet
- Patent Literature 2:: International Patent Application WO2005/21564A1 Pamphlet
- Patent Literature 3:: International Patent Application WO2006/35725A1 Pamphlet
- Patent Literature 4:: Japanese Patent Kokai No. H06-0197783
- Patent Literature 5:: Japanese Patent Kokai No. 2004-166624
- Patent Literature 6:: Japanese Patent Kokai No. 2005-192510

### Non-Patent Literature

Non-Patent Literature 1: Journal of American Chemical Society, USA, (1949), Vol. 71, pp. 353-358
Non-Patent Literature 2: Biosci. Biotech. Biochem., Vol. 57, pp. 1225-1227 (1993)
Non-Patent Literature 3: Biosci. Biotech. Biochem., Vol. 72, pp. 3277-3280 (2008)

### DISCLOSURE OF INVEVTION

### Object of the Invention

Objects of the present invention are to provide a novel cyclic saccharide composed of D-glucose as constituents, a novel enzyme forming the cyclic saccharide, processes for producing the same, and uses thereof.

### Mean to Attain the Object

To solve the above objects, the present inventors have extensively screened microorganisms capable of producing a novel enzyme which forms a novel cyclic saccharide when allowed to act on dextran or its partial hydrolysate, having α-1,6 glucan, a glucose polymer which is formed by binding D-glucose molecules in a linear fashion *via* α-1,6 glucosidic linkages, as a basic structure.

As the results, the present inventors found that a strain D1110 belonging to the genus *Agreia* and a strain D2006 belonging to the genus *Microbacterium,* which are newly isolated from soil samples, produce novel enzymes, and also found that a remarkable amount of a novel saccharide having a structure of binding four D-glucose molecules to form cyclic structure *via* α-1,6 glucosidic linkages (hereinafter, simply abbreviated as "α-1,6 linkage"), can be produced by the action of the novel enzyme from α-1,6 glucan, or dextran and its partial hydrolysate, having the α-1,6 glucan as the basic structure.

The present inventors have cultured the microorganisms having the ability of producing the novel enzyme which forms the cycloisomaltotetraose, purified the cycloisomaltotetraose-forming enzyme from the culture and revealed its properties, and then established a process for producing the same. Further, the present inventors also established a DNA encoding the enzyme, a recombinant DNA containing the DNA, and transformant comprising the recombinant DNA. Furthermore, the present inventors also established a process for producing cycloisomaltotetraose or a saccharide composition containing the same by using the enzyme and uses thereof, and then completed the present invention.

The present invention solves the above objects by providing a novel saccharide, cycloisomaltotetraose having a structure of which four D-glucose molecules are bound in cyclic form *via* α-1,6 glucosidic linkages, a novel cycloisomaltotetraose-forming enzyme which forms the saccharide, a DNA encoding the enzyme, a recombinant DNA comprising the DNA and a transformant having the recombinant DNA, processes for producing those, a composition comprising cycloisomaltotetraose or a saccharide composition comprising the same such as foods and beverages, luxury grocery items, feeds, baits, cosmetics, pharmaceuticals, industrial supplies, etc.

### Effect of the Invention

According to the present invention, cycloisomaltotetraose, a novel cyclic saccharide which has not been ever known, and a saccharide composition comprising the same can be provided, and the present invention enables the use of those in various fields including foods and beverages, cosmetics, and pharmaceuticals.

### BRIEF DESCRIPTION OF DRAWINGS

FIG. 1 shows a TLC chromatogram of the enzyme reaction mixture obtained by allowing a crude enzyme from a microorganism strain D1110 to act on dextran as a substrate.
FIG. 2 shows a HPLC chromatogram of the purified preparation of "Unknown Saccharide I".
FIG. 3 shows a ¹H-NMR spectrum of the purified preparation of "Unknown Saccharide I".
FIG. 4 shows a ¹H-¹H COSY spectrum of the purified preparation of "Unknown Saccharide I".
FIG. 5 shows a ¹³C-NMR spectrum of the purified preparation of "Unknown Saccharide I".
FIG. 6 shows a HSQC spectrum of the purified preparation of "Unknown Saccharide I".
FIG. 7 shows a HMBC spectrum of the purified preparation of "Unknown Saccharide I".
FIG. 8 shows the structure of cycloisomaltotetraose of the present invention.
FIG.9 shows a micrograph of crystals of cycloisomaltotetraose (Magnification: x 400).
FIG. 10 shows a powder X-ray diffraction pattern of crystalline cycloisomaltotetraose pentahydrate.
FIG. 11 shows the optimum temperature of cycloisomaltotetraose-forming enzyme from *Agreia* sp. D1110.
FIG. 12 shows the optimum pH of cycloisomaltotetraose-forming enzyme from *Agreia* sp. D1110.
FIG. 13 shows the thermal stability of cycloisomaltotetraose-forming enzyme from *Agreia* sp. D1110.
FIG. 14 shows the pH stability of cycloisomaltotetraose-forming enzyme from *Agreia* sp. D1110.
FIG. 15 shows the optimum temperature of cycloisomaltotetraose-forming enzyme derived from *Microbacterium trichothecenolyticum* D2006.
FIG. 16 shows the optimum pH of cycloisomaltotetraose-forming enzyme from *Microbacterium trichothecenolyticum* D2006.
FIG. 17 shows the thermal stability of cycloisomaltotetraose-forming enzyme from *Microbacterium trichothecenolyticum* D2006.
FIG. 18 shows the pH stability of cycloisomaltotetraose-forming enzyme from *Microbacterium trichothecenolyticum* D2006.
FIG. 19 shows the homology between amino acid sequences encoded by ORF9038 and ORF5328, genes of cycloisomaltotetraose-forming enzymes from *Agreia* sp. D1110 and *Microbacterium trichothecenolyticum* D2006, respectively.
FIG. 20 shows a recombinant DNA, pRSET A-ORF9038. In the figure, the black bold line means a DNA encoding cycloisomaltotetraose-forming enzyme from *Agreia* sp. D1110.
FIG. 21 shows a HPLC chromatogram of a reaction mixture obtained by allowing a supernatant of cell extract prepared by culturing a recombinant E. *coli* E9038 having the recombinant DNA, pRSET A-ORF9038 to act on dextran as a substrate.
FIG. 22 shows a HPLC chromatogram of a saccharide composition containing glucosyl-derivatives of cycloisomaltotetraose, obtained by the steps of allowing cyclomaltodextrin glucanotransferase to act on a glucosyl donor, α-cyclodextrin in a presence of an acceptor, cycloisomaltotetraose for glycosyl transferring reaction, hydrolyzing the resulting products by glucoamylase, and subjecting the resulting reaction mixture to alkaline treatment.
FIG. 23 shows the structures of two glucosyl-derivatives of cycloisomaltotetraose.

### MODE FOR CARRYING OUT THE INVENTION

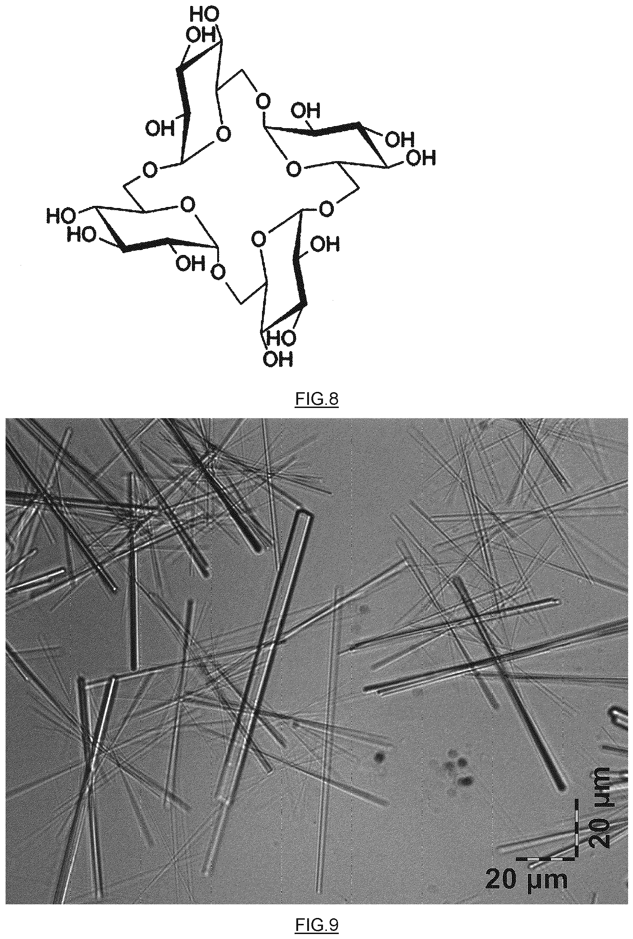

The present invention relates to a novel cyclic saccharide, cycloisomaltotetraose. Cycloisomaltotetraose as referred to in the present invention means a cyclic saccharide having a structure in which four D-glucose molecules are bound in cyclic form *via* α-1,6 glucosidic linkages, i.e., *cyclo*{→6)-α-D-glucopyranosyl-(1→6)-α-D-glucopyranosyl-(1→6)-α-D-glucopyranosyl-(1→6)-α-D-glucopyranosyl-(1→} (See FIG. 8).

Cycloisomaltotetraose of the present invention is a novel and ever unknown saccharide, firstly found by the present inventors in a reaction mixture obtained by allowing a culture broth of a microorganism isolated from a soil as a crude enzyme to act on a substrate, dextran. The present invention encompasses cycloisomaltotetraose independently by its source and process for producing it, as far as it has the above structure.

Cycloisomaltotetraose of the present invention encompasses that in a crystalline form as another embodiment. The crystal of cycloisomaltotetraose of the present invention is, usually, in the form of pentahydrate crystal.

The crystalline cycloisomaltotetraose pentahydrate of the present invention shows characteristic diffraction peaks at the diffraction angles (2*θ*) of 9.0°, 9.8°, 11.8°, 13.1°, and 19.1° in its powder X-ray diffraction pattern.

The present invention includes, as another embodiment, a saccharide composition comprising cycloisomaltotetraose which comprises cycloisomaltotetraose and/or crystalline cycloisomaltotetraose together with other saccharides. The form of the saccharide composition comprising cycloisomaltotetraose may be any of a syrup, powder, powder containing crystals or solid.

The present invention includes, as another embodiment, a glucosyl derivative of cycloisomaltotetraose having a structure in which one or two D-glucose molecules are bound to cycloisomaltotetraose. The binding mode of D-glucose to cycloisomaltotetraose in the glucosyl derivative is not particularly restricted, and may be any of α-1,2, α-1,3, or α-1,4 linkage. A glucosyl derivatives in which one D-glucose molecule is bound to one glucose residue of the four glucose residues constituting the cycloisomaltotetraose *via* α-1,3 linkage is present in trace amounts in the reaction mixture obtained by cycloisomaltotetraose-forming reaction of the enzyme described later. Further, as illustrated in the examples below, glucosyl derivatives of cycloisomaltotetraose can be produced and collected by the steps of allowing an appropriate glycosyltransferase to act on an appropriate glycosyl donor in the presence of cycloisomaltotetraose as an acceptor and enzymatically hydrolyzing the resulting transglycosylation product. As the glycosyltransferase, for example, α-glucosidase, β-glucosidase, α-amylase, cyclomaltodextrin glucanotransferase (CGTase), etc. can be used. For the enzymatic hydrolysis of the transglycosylation product, for example, glucoamylase, β-amylase, etc. can be used. Furthermore, by selecting the glycosyl donor and the glycosyltransferase, glycosyl derivatives of cycloisomaltotetraose in which a saccharide other than D-glucose is transferred to cycloisomaltotetraose, for example, fructosyl derivatives, galactosyl derivatives, etc. can also be advantageously produced and collected.

Cycloisomaltotetraose-forming enzyme as referred to in the present invention means any enzyme which acts on α-1,6 glucan with a glucose polymerization degree of 4 or higher, dextran, or partial dextran hydrolysate and catalyzes the reaction of forming cycloisomaltotetraose. Cycloisomaltotetraose-forming enzyme of the present invention encompasses any enzyme catalyzing the above reaction without being restricted by its source, form, and purity, and by the reaction mechanism of acting to α-1,6 glucan having a glucose polymerization degree of 4 or higher, dextran or partial dextran hydrolysate and forming cycloisomaltotetraose. For example, when cycloisomaltotetraose-forming enzyme of the present invention is allowed to act on dextran, it is presumed that the enzyme cleaves α-1,6 linkages of dextran in isomaltotetraose units, and catalyzes an intramolecular α-1,6 transglycosylation of transferring the C1 position of the reducing end glucose of the isomaltotetraose to the hydroxyl group at the C6 position of the non-reducing end glucose of the same molecule to form cycloisomaltotetraose by cyclization. In a preferred example, cycloisomaltotetraose-forming enzyme of the present invention also has an activity of further acting on the once formed cycloisomaltotetraose and hydrolyzing to cleave the cyclic structure to form isomaltotetraose.

The enzyme activity of cycloisomaltotetraose-forming enzyme of the present invention can be assayed as follows:
A substrate solution is prepared by dissolving dextran or partial dextran hydrolysate in 20 mM acetate buffer (pH 6.0) to give a concentration of 1.11% (w/v). To 0.9 mL of the substrate solution, 0.1 mL of an enzyme solution, which prepared by 20 mM acetate buffer solution (pH 6.0) is added, and the mixture solution is incubated at 40°C to start the reaction. Then, 0.4 mL each of the reaction mixture is sampled at reaction times of 0.5 min and 30.5 min, and the reaction is stopped by heating at 100°C for 10 min for inactivate the enzyme. Successively, the amount of cycloisomaltotetraose in each reaction mixture is determined by HPLC described later in Experiment 1, and then the amount of cycloisomaltotetraose formed in 30 min-reaction is calculated by subtracting the amount of cycloisomaltotetraose at 0.5 min-reaction from that at 30.5 min-reaction. One unit activity (U) of cycloisomaltotetraose-forming enzyme is defined as the amount of enzyme which forms one µmole of cycloisomaltotetraose per minute under the above conditions.

As a concrete example of cycloisomaltotetraose-forming enzyme of the present invention, the enzyme having the following physicochemical properties can be listed.
(1) Molecular weight
   86,000 ± 10,000 daltons when determined on SDS-polyacrylamide gel electrophoresis;
(2) Optimum temperature
   35 to 40°C when reacted at pH 6.0 for 30 min;
(3) Optimum pH
   pH 5.5 to 6.0 when reacted at 40°C for 30 min;
(4) Thermal stability
   Stable up to 40°C when incubated at pH 6.0 for 30 min; and
(5) pH Stability
   Stable in a pH range of 5.0 to 10.0 when incubated at 4°C for 16 hours;

As another concrete example of cycloisomaltotetraose-forming enzyme of the present invention, the enzyme having the following physicochemical properties can be listed.
(1) Molecular weight
   86,000 ± 10,000 daltons when determined on SDS-polyacrylamide gel electrophoresis;
(2) Optimum temperature
   40°C when reacted at pH 6.0 for 30 min;
(3) Optimum pH
   About pH 5.5 when reacted at 40°C for 30 min;
(4) Thermal stability
   Stable up to 35°C when incubated at pH 6.0 for 30 min; and
(5) pH Stability
   Stable in a pH range of 5.0 to 7.0 when incubated at 4°C for 18 hours;

Cycloisomaltotetraose-forming enzyme of the present invention, having the above physicochemical properties, may have an amino acid sequence of SEQ ID NO: 5 or SEQ ID NO: 6 as the N-terminal amino acid sequence.

However, cycloisomaltotetraose-forming enzyme having the above physicochemical properties or above N-terminal amino acid sequence is only an example, and enzymes having different physicochemical properties or different N-terminal amino acid sequences as described above are also encompassed in cycloisomaltotetraose-forming enzyme of the present invention as long as they form cycloisomaltotetraose.

Usually, cycloisomaltotetraose-forming enzyme of the present invention has a prescribed amino acid sequence. For example, amino acid sequences of SEQ ID NO: 7, SEQ ID NO: 8, or those homologous to SEQ ID NO: 7 or SEQ ID NO: 8 can be listed. A variant enzyme having an amino acid sequence homologous to SEQ ID NO: 7 or SEQ ID NO: 8 means an enzyme having an amino acid sequence where one or more amino acids in SEQ ID NO: 7 or SEQ ID NO: 8 are deleted, replaced or added with other amino acids without altering the enzyme activity of acting on dextran or partial dextran hydrolysates and forming cycloisomaltotetraose. As such a variant enzyme, it is preferable that the enzyme has an amino acid sequence with a homology to SEQ ID NO: 7 or SEQ ID NO: 8 of, usually, 70% or higher, desirably, 80% or higher, more desirably, 90% or higher.

Although cycloisomaltotetraose-forming enzyme of the present invention is not restricted by its source, microorganisms are preferable as the source. Particularly, a microorganism, strain D1110, isolated by the present inventors from a soil sample or mutants thereof, or a microorganism, strain D2006, also isolated from a soil sample, or mutant thereof can be preferably used. The mutants as referred to as in the present invention means, for example, mutants whose culture properties are improved than the parent strain, enzyme-hyper-producing mutants whose ability of producing cycloisomaltotetraose-forming enzymes are improved than the parent strain, or mutants producing cycloisomaltotetraose-forming enzyme with higher enzymatic activity, created by artificially introducing the mutation into the parent strain.

As described above, the microorganisms, strain D1110 and strain D2006, having the ability to produce cycloisomaltotetraose-forming enzyme are microorganisms newly isolated from the soil by the present inventors. As described later in Experiment 4, strain D1110 and strain D2006 were identified as *Agreia* sp. and *Microbacterium trichothecenolyticum,* respectively, by identifying the bacterial genus and species based on the homologies of the nucleotide sequences of 16S rRNA (16S rDNA) with those of known bacteria. Based on these results, the present inventors named those novel microorganisms as *"Agreia* sp. D1110" and *"Microbacterium trichothecenolyticum* D2006" and deposited those on December 28, 2018, in National Institute of Technology and Evaluation (NITE), NITE Patent Microorganisms Depositary (NPMD), 2-5-6 Kazusakamatari, Kisarazu-shi, Chiba, Japan, and accepted under the accession numbers of NITE BP-02815 and NITE BP-02816, respectively.

The microorganism having an ability of producing cycloisomaltotetraose-forming enzyme of the present invention includes the above bacterial strains and those mutants, and microorganisms having an ability of producing cycloisomaltotetraose-forming enzyme, belonging other genus and species isolated and selected from nature by the screening comprising the steps of allowing the culture broth as a crude enzyme to act on the substrate dextran and investigating the formation of cycloisomaltotetraose, and those mutants.

The present invention also relates to the DNAs comprising the nucleotide sequences which encodes the amino acid sequences of cycloisomaltotetraose-forming enzymes of the present invention described above, and complement nucleotide sequences to the nucleotide sequences also described above. The DNA of the present invention includes any DNA originated from the nature and those which are synthesized artificially as far as they encode the amino acid sequence of cycloisomaltotetraose-forming enzyme. For example, microorganisms of the genus *Agreia* including *Agreia* sp. D1110, and microorganisms of the genus *Microbacterium* including *Microbacterium trichothecenolyticum* D2006 can be used as the natural sources of the DNA. A genomic DNA containing the DNA of the present invention can be obtained from the cells of these microorganisms. A genomic DNA containing the DNA can be released extracellularly by the steps of inoculating any of the microorganisms into a nutrient medium, culturing about 5 to 10 days under aerobic conditions, collecting the cells from the culture, and treating the cells with cell-lytic enzymes such as lysozyme and β-glucanase or with ultrasonication. In addition to the methods described above, use of protein-hydrolyzing enzymes such as proteinases, detergents such as SDS, and freeze-thaw method are also applicable. The objective genomic DNA can be obtained from the cells treated by using conventional methods in the art, for example, such as phenol-extraction, alcohol-precipitation, centrifugation, and ribonuclease-treatment. To artificially synthesize the DNA of the present invention, it can be chemically synthesized based on the amino acid sequence described together with the nucleotide sequence of SEQ ID NO: 9 or SEQ ID NO: 10. PCR-method is also applicable to obtain the DNA by using a genomic DNA containing the DNA as a template and an appropriate chemically synthesized DNA as a primer.

The DNA of the present invention, for example, includes a DNA having a nucleotide sequence of SEQ ID NO: 9 or SEQ ID NO: 10, a DNA having a homologous nucleotide sequence to those, and a DNA having a complement nucleotide sequence to those. A DNA having a homologous nucleotide sequence to SEQ ID NO: 9 or SEQ ID NO: 10, means that having a nucleotide sequence where one or more nucleotides in SEQ ID NO: 9 or SEQ ID NO: 10 are deleted, replaced or added without altering the activity of cycloisomaltotetraose-forming enzyme encoded thereby. The homology (sequence identity) of nucleotide sequence to SEQ ID NO: 9 or SEQ ID NO: 10 of such a variant DNA is preferable to be, usually, 70% or higher, desirably, 80% or higher, more desirably, 90% or higher, and most desirably, 95% or higher. The DNA of the present invention encompasses a DNA having a nucleotide sequence where one or more nucleotides of SEQ ID NO: 9 or SEQ ID NO: 10 are replaced with other nucleotides without altering the encoded amino acid sequence based on the degeneracy of genetic code, and their complement nucleotide sequence.

A recombinant DNA can be advantageously obtained by inserting the DNA of the present invention into an appropriate autonomously replicable vector. A recombinant DNA is usually composed of an objective DNA and an autonomously replicable vector, and if an objective DNA can be obtained, it can be easily prepared by conventional recombinant DNA techniques. As examples of such vectors, plasmids, phage or cosmid vectors, etc., can be used and appropriately selected depending on the host cell to be introduced or the introduction method. Types of vectors are not specifically restricted, and that possible to be expressed in a host cell can be appropriately selected. Depending on the type of the host cell, a promoter sequence can be appropriately selected to express the gene, and a vector constructed by incorporating the promoter sequence and the gene into various plasmids can be used as the expression vectors. As such expression vectors, for example, phage vectors, plasmid vectors, viral vectors, retroviral vectors, chromosome vectors, episomal vectors and virus-derived vectors (e.g., bacterial plasmid, bacteriophage, yeast episomes, yeast chromosome elements) and viruses (e.g., baculovirus, papovavirus, vaccinia virus, adenovirus, tripox virus, pseudorabies virus, herpes virus, lentivirus and retrovirus) and vectors derived from their combinations (e.g., cosmid and phagemid) are available.

Preferred vectors for use in bacteria include, for example, pRSET A, pQE-70, pQE-60, pBS vectors, Phagescript vectors, Bluescript vectors, pNH8A, pNH6a, pNH18A and pNH46A; and ptrc99a, pKK223-3, pKK233-3, pDR540, pRIT5, and the like. Preferred vectors for use in eukaryotic organisms include, for example, pWLNE0, pSV2CAT, pOG44, pXT1 and pSG; and pSVK3, pBPV, pMSG, pSVL and the like.

To insert the DNA of the present invention into such vectors, conventional methods used in the field are usually employed. Specifically, the gene DNA containing the objective DNA and an autonomously replicable vector are firstly cleaved by restriction enzymes and/or ultrasonic, then the formed DNA fragment and the vector fragment are ligated. A recombinant DNA thus obtained can be replicated infinitely by the steps of introducing it into an appropriate host and culturing the resulting transformant.

The recombinant DNA, thus obtained, can be introduced into an appropriate host microorganism including *E. coli, Bacillus subtilis, Actinomycetes,* yeast, etc. To obtain a transformant, a colony hybridization method can be applied. Also, a transformant can be selected by the steps of culturing the microorganism in nutrient medium and detecting the transformant producing the cycloisomaltotetraose-forming enzyme.

Any nutrient culture medium can be used for cultivating microorganisms, including a transformant, capable of producing cycloisomaltotetraose-forming enzyme of the present invention as long as they can grow therein and produce cycloisomaltotetraose-forming enzyme: for example, synthetic- and natural-culture media can be used as nutrient culture media. Any carbon source can be used as long as it is utilized for the growth by the microorganisms: examples of such carbon source are saccharides such as dextran and its partial hydrolysates, starch and phytoglycogen, obtainable from plants; glycogen and pullulan, obtainable from animals and microorganisms; hydrolysates thereof, glucose, fructose, lactose, sucrose, mannitol, sorbitol, and saccharide syrups; and organic acids such as citric acid and succinic acid. The concentrations of these carbon sources in nutrient culture media can be appropriately chosen. The nitrogen sources usable for the cultivation are, for example, inorganic nitrogen compounds such as ammonium salts and nitrates; organic nitrogen compounds such as urea, corn steep liquor, casein, peptone, yeast extract and meat extract. The inorganic ingredients usable in the cultivation are, for example, calcium salts, magnesium salts, potassium salts, sodium salts, phosphates, manganese salts, zinc salts, iron salts, copper salts, molybdate salts, and cobalt salts. In addition, if necessary, amino acids and vitamins can be suitably used.

The microorganisms of the present invention can be cultured under aerobic conditions, usually, at a temperature in the range of 15-37°C and at a pH in the range of 5.5-10, preferably, at a temperature in the range of 20-34°C and at a pH in the range of 5.5-8.5. The cultivation time can be set to a time longer than that required for the growth of the microorganisms, preferably, 10-150 hours. The concentration of dissolved oxygen is not specifically restricted, but usually set to 0.5-20 ppm. The concentration of dissolved oxygen can be kept within the above range by controlling aeration and agitation. The cultivation can be carried out in a batch-wise or a continuous manner.

After culturing the microorganisms capable of producing cycloisomaltotetraose-forming enzyme, according to the method described above, the culture broth containing the enzyme of the present invention is collected. The major activity of cycloisomaltotetraose-forming enzyme is found in the cell-free supernatant. Both the cell-free supernatant and the culture broth can be used as a crude enzyme. In order to remove cells from the culture broth, conventional liquid-solid separation methods can be used. For example, methods to directly centrifuge the resultant culture broth, as well as those to filtrate the culture broth with pre-coated filters or to separate cells by membrane filtration using plane filters or follow fibers, can be suitably used. Although the cell-free supernatant thus obtained can be used intact as crude enzyme solution, it is generally used after concentration. The concentration methods usable in the invention are, for example, salting out using ammonium sulfate, sedimentation using acetone or alcohol, and concentration using membranes such as plane filters or follow fibers.

Furthermore, cycloisomaltotetraose-forming enzyme can be subjected to conventional immobilization using the cell-free supernatant and the concentrate. Examples of such conventional methods are conjugation methods using ion exchangers, covalent bindings and adsorptions using resins and membranes, and inclusion methods using high molecular weight substances.

As described above, a crude enzyme solution can be used intact or after concentrating it as cycloisomaltotetraose-forming enzyme of the present invention. If necessary, cycloisomaltotetraose-forming enzyme can be advantageously used after separating or purifying the crude enzyme solution by suitable conventional methods used in the art. For example, a purified cycloisomaltotetraose-forming enzyme preparation exhibiting an electrophoretically single band can be obtained by the steps of salting out a cell-free supernatant with ammonium sulfate, dialyzing the resulting crude enzyme preparation, and successively purifying the dialyzed enzyme preparation on anion-exchange column chromatography using "DEAE-TOYOPEARL 650S", an anion-exchange gel commercialized Tosoh Corporation, Tokyo, Japan, and hydrophobic chromatography using "PHENYL-TOYOPEARL 650M", a hydrophobic gel commercialized Tosoh Corporation, Tokyo, Japan.

When cycloisomaltotetraose-forming enzyme is a recombinant enzyme, the enzyme may be accumulated intracellularly, depending on the kinds of host microorganisms. In such case, while the cells or the culture broth can be used intact, the recombinant enzyme can be advantageously used after extracting it from cells by using osmotic-shock methods or detergents, or by disrupting cells using ultrasonication methods or cell-wall digesting enzymes, and separating it from the cells or cell debris.

By using the native or recombinant cycloisomaltotetraose-forming enzyme obtained by the above method, it is possible to produce cycloisomaltotetraose of the present invention, and a saccharide composition containing cycloisomaltotetraose.

In producing cycloisomaltotetraose and a saccharide composition containing cycloisomaltotetraose, α-1,6 glucans such as dextran and partial dextran hydrolysate obtainable by partially hydrolyzing dextran using acids or dextran-hydrolyzing enzyme, dextranase, can be used as a substrate material for the reaction of cycloisomaltotetraose-forming enzyme. Further, as described later in Experiment 14, since cycloisomaltotetraose-forming enzyme of the present invention acts on isomaltotetraose and forms cycloisomaltotetraose, it is revealed that α-1,6 glucans having a glucose polymerization degree of at least 4 can be used as substrates of the enzyme. Furthermore, since the branched α-glucan disclosed in WO2008/136331 pamphlet has an α-1,6 glucan structure that D-glucose molecules are bound *via* α-1,6 linkages as the partial structure, it can be used as a substrate material for the action of cycloisomaltotetraose-forming enzyme.

When cycloisomaltotetraose-forming enzyme is allowed to act on a substrate, the substrate concentration is not specifically restricted. For example, when using an aqueous substrate solution with a relatively high concentration of 10 % (w/w) or more, the reaction by cycloisomaltotetraose-forming enzyme of the present invention is difficult to proceed, therefore, the substrate concentration of 5 %(w/w) or lower is preferred, and under this condition, cycloisomaltotetraose can be advantageously produced. The reaction temperature used in the present enzymatic reaction can be set to a temperature at which the reaction proceeds, i.e. a temperature up to about 55°C, preferably, a temperature in the range of 30 to 45°C. The reaction pH is controlled in the range of, usually, 4.0 to 8.0, preferably, 4.5 to 7.5. Since the amount of enzyme and the reaction time are closely related, the conditions are adequately chosen with respect to the progress of the objective enzymatic reaction.

As described above, cycloisomaltotetraose-forming enzyme of the present invention forms cycloisomaltotetraose from α-1,6 glucan having a glucose polymerization degree of 4 or higher, dextran, or a saccharide having α-1,6 glucan structure as a partial structure. Therefore, cycloisomaltotetraose can be produced by using starch or partial starch hydrolysate as a substrate material when cycloisomaltotetraose-forming enzyme of the present invention and an enzyme, having an activity of forming α-1,6 glucan having a glucose polymerization degree of 4 or higher, dextran, or a saccharide having α-1,6 glucan structure as a partial structure from starch or partial starch hydrolysates, are used in combination. For example, dextrin dextranase can be used as the enzyme that acts on starch or partial starch hydrolysates to form α-1,6 glucan having a glucose polymerization degree of 4 or higher and dextran, and an α-glucosyltransferase disclosed in WO2008/136331 pamphlet can be used as the enzyme having an activity of forming a saccharide having α-1,6 glucan structure as a partial structure.

In producing cycloisomaltotetraose from starch or partial starch hydrolysates by a combination of the enzymes described above, a sequential reaction of firstly forming α-1,6 glucans having a glucose polymerization degree of 4 or higher, dextran, or saccharides having an α-1,6 glucan structure as a partial structure by allowing an enzyme which acts on starch or partial starch hydrolysates and forms α-1,6 glucans having a glucose polymerization degree of 4 or higher, dextran, or saccharides having an α-1,6 glucan structure as a partial structure to act on starch or partial starch hydrolysates, and successively forming cycloisomaltotetraose by allowing cycloisomaltotetraose-forming enzyme of the present invention to act on the resulting products, can be selected. Also, a simultaneous reaction of allowing an enzyme which acts on starch or partial starch hydrolysates and forms α-1,6 glucans having a glucose polymerization degree of 4 or higher, dextran, or saccharides having an α-1,6 glucan structure as a partial structure to act on starch or partial starch hydrolysates and cycloisomaltotetraose-forming enzyme of the present invention to act on starch or partial starch hydrolysates simultaneously, can be selected.

The reaction mixture, thus obtained by the above reaction, can be used intact as a saccharide solution comprising cycloisomaltotetraose. Optionally, the saccharide solution comprising cycloisomaltotetraose can be used after hydrolyzing the concomitant isomaltooligosaccharides by allowing dextranase, α-glucosidase, or glucoamylase to act on the saccharide comprising cycloisomaltotetraose. Further, the saccharide comprising cycloisomaltotetraose can be arbitrary processed by hydrogenating to convert reducing saccharides remained in the reaction mixture into sugar alcohols for eliminating the reducing power. Usually, a saccharide solution comprising cycloisomaltotetraose is used after further purification. Conventional methods used for purifying saccharides can be arbitrarily selected as the purification method. For example, one or more purification methods selected from the group consisting of decoloring with an activated charcoal; desalting with ion exchange resins in H and OH form; fractionation by column chromatography such as ion exchange column chromatography, charcoal column chromatography, and silica gel column chromatography; separation using organic solvents such as alcohol and acetone; separation using a membrane having a suitable separability; fermentation using microorganisms, which utilize and decompose concomitant saccharides but not utilize cycloisomaltotetraose, such as yeasts; and eliminating the remaining reducing sugars with alkaline treatments; can be arbitrarily used.

More particularly, ion exchange column chromatography can be suitably used as an industrial-scale preparation of the objective saccharides. The objective cycloisomaltotetraose or a saccharide composition comprising the same with an improved purity can be advantageously prepared by, for example, column chromatography using a strong acidic cation exchange resin as described in Japanese Patent Kokai Nos. 23,799/83 and 72,598/83 to remove concomitant saccharides. In this case, any one of fixed bed, moving bed, and semi-moving bed methods can be employed.

A solution comprising cycloisomaltotetraose thus obtained or a saccharide solution with an improved cycloisomaltotetraose content contains cycloisomaltotetraose in an amount of, usually, 10% (w/w) or higher, desirably, 40% (w/w) or higher, on a dry solid basis (hereinafter, abbreviated as "d.s.b."), and is usually concentrated into a product in a syrupy form. The syrupy product can be arbitrarily dried to make into an amorphous solid product or an amorphous powder product.

Furthermore, by purifying the aqueous saccharide solution comprising cycloisomaltotetraose by conventional methods, a cycloisomaltotetraose high-content saccharide and a cycloisomaltotetraose preparation with a high purity can be obtained. In addition, crystals of cycloisomaltotetraose and a particulate composition comprising crystalline cycloisomaltotetraose can be prepared by the crystallization using the cycloisomaltotetraose high-content saccharide or the cycloisomaltotetraose preparation with a high purity as a material.

In addition, cycloisomaltotetraose of the present invention is only slightly hydrolyzed by small intestinal mucosal α-glucosidase. Therefore, cycloisomaltotetraose is a hardly digestible saccharide that not digested and adsorbed when orally ingested, and is considered to be a saccharide with no toxicity and no harm.

Cycloisomaltotetraose of the present invention and the saccharide compositions comprising the same can be advantageously used as a sweetener, taste-improving agent, quality-improving agent, stabilizer, color-deterioration preventing agent, excipient, base agent for powderization, etc., for various compositions such as foods and beverages, luxury grocery items, feeds, baits, cosmetics, pharmaceuticals, and industrial products in combination with other ingredients.

Cycloisomaltotetraose of the present invention and the saccharide compositions comprising the same can be used intact as a seasoning for sweetening products. If necessary, they can be advantageously used in combination with other sweeteners, for example, powdery syrup, glucose, isomerized sugar, sucrose, maltose, trehalose, honey, maple sugar, sorbitol, maltitol, dihydrochalcone, stevioside, α-glycosyl stevioside, sweetener of *Momordica grosvenori,* glycyrrhizin, thaumatin, sucralose, L-aspartyl L-phenylalanine methyl ester, saccharine, glycine and alanine; and fillers such as dextrin, starch, and lactose.

Further, powdery products of cycloisomaltotetraose of the present invention and the saccharide compositions comprising the same can be arbitrarily used intact or, if necessary, after mixing with fillers, excipients, binders, etc., and then shaped into various shapes such as granules, spheres, sticks, plates, cubes, and tablets.

Cycloisomaltotetraose of the present invention and the saccharide compositions comprising the same have sweetness which well harmonizes with other materials having sour-, salty-, astringent-, *umami-,* and bitter-taste; and cycloisomaltotetraose of the present invention has a high acid- and heat-tolerance as described later in Experiments. Thus, they can be advantageously used to sweeten and/or improve the taste and quality of general foods and beverages.

Cycloisomaltotetraose of the present invention and the saccharide compositions comprising the same can be advantageously used as a sweetener, taste-improving agent, and quality-improving agent for various seasonings such as a soy sauce, powdered soy sauce, *miso, "funmatsu-miso"* (a powdered *miso), "moromi"* (a refined sake), *"hishio"* (a refined soy sauce), *"furikake"* (a seasoning powder for rice), mayonnaise, dressing, vinegar, *"sanbai-zu"* (a sauce of sugar, soy sauce and vinegar), *"funmatsu-sushi-zu"* (powdered vinegar for sushi), *"chuka-no-moto"* (an instant mix for Chinese dish), *"tentsuyu"* (a sauce for Japanese deep fat fried food), *"mentsuyu"* (a sauce for Japanese vermicelli), sauce, catsup, *"yakiniku-no-tare"* (a sauce for Japanese grilled meat), curry roux, instant stew mix, instant soup mix, *"dashi-no-moto"* (an instant stock mix), mixed seasoning, *"mirin"* (a sweet sake), *"shin-mirin"* (a synthetic *mirin*), table sugar, and coffee sugar. Also, cycloisomaltotetraose and the saccharide compositions comprising the same can be advantageously used to sweeten and to improve the taste and quality of various *"wagashi"* (Japanese confectionaries) such as *"senbei"* (a rice cracker), *"arare"* (a fried rice cake cube), *"okoshi"* (a millet and rice cake), *"gyuhi"* (a starch paste), *"mochi"* (a rise cake) and the like, *"manju"* (a bun with a bean-jam), *"uiro"* (a sweet rice jelly), *"an"* (a bean-jam) and the like, *"yokan"* (a sweet jelly of beans), *"mizu-yokan"* (a soft azuki-bean jelly), *"kingyoku"* (a kind of *yokan),* jelly, pao de Castella, and *"amedama"* (a Japanese toffee); Western confectioneries such as a bun, biscuit, cracker, cookie, pie, pudding, butter cream, custard cream, cream puff, waffle, sponge cake, doughnut, chocolate, chewing gum, caramel, nougat, and candy; frozen desserts such as an ice cream and sherbet; syrups such as a *"kajitsu-no-syrup-zuke"* (a preserved fruit) and *"korimitsu"* (a sugar syrup for shaved ice); pastes such as a flour paste, peanut paste, and fruit paste; processed fruits and vegetables such as a jam, marmalade, *"syrup-zuke"* (fruit pickles), and *"toka"* (candied fruits); pickles and pickled products such as a *"fukujin-zuke"* (red colored radish pickles), *"bettara-zuke"* (a kind of whole fresh radish pickles), *"senmai-zuke"* (a kind of sliced fresh radish pickles), and *"rakkyo-zuke"* (pickled shallots); premix for pickles and pickled products such as a *"takuan-zuke-no-moto"* (a premix for pickled radish), and *"hakusai-zuke-no-moto"* (a premix for fresh white rape pickles); meat products such as a ham and sausage; products of fish meat such as a fish ham, fish sausage, *"kamaboko"* (a steamed fish paste), *"chikuwa"* (a kind of fish paste), and *"tenpura"* (a Japanese deep-fat fried fish paste); *"chinmi"* (relish) such as a *"uni-no-shiokara"* (salted urchin), *"ika-no-shiokara"* (salted squid), *"su-konbu"* (vinegared kelp), *"saki-surume"* (dried squid strips), *"fugu-no-mirin-boshi"* (a dried *mirin*-seasoned swellfish), seasoned fish flour such as of Pacific cod, sea bream, shrimp, etc.; *"tsukudani"* (foods boiled down in soy sauce) such as those of laver, edible wild plants, dried squid, small fish, and shellfish; daily dishes such as a *"nimame"* (cooked beans), potato salad, and *"konbu-maki"* (a sea tangle roll); milk products; canned and bottled products such as those of meat, fish meat, fruit, and vegetable; alcoholic beverages such as a synthetic *sake*, fermented liquor, *sake*, fruit liquor, low-malt beer and beer; soft drinks such as a coffee, cocoa, juice, carbonated beverage, sour milk beverage, and beverage containing a lactic acid bacterium; instant food products such as instant pudding mix, instant hot cake mix, instant juice, instant coffee, *"sokuseki-shiruko"* (an instant mix of *azuki-bean* soup with rice cake), and instant soup mix; and other foods and beverages such as solid foods for babies, foods for therapy, drinks, peptide foods, and frozen foods.

Cycloisomaltotetraose and the saccharide compositions comprising the same can be arbitrarily used to improve the taste preference of feeds and pet foods for animals and pets such as domestic animals, poultry, honey bees, silk warms, and fishes; and also they can be advantageously used as a sweetener and taste-improving agent, taste-curing agent, quality-improving agent, and stabilizer for various compositions including luxury grocery items, cosmetics, pharmaceuticals, quasi drugs in a solid, paste or liquid form such as tobacco, cigarette, tooth paste, lipstick, rouge, lip cream, internal liquid medicine, tablet, troche, cod-liver oil in the form of drop, oral refrigerant, cachou, and gargle.

When used as a quality-improving agent or stabilizer, cycloisomaltotetraose and the saccharide compositions comprising the same can be advantageously used in biologically active substances susceptible to lose their effective ingredients and activities, as well as in health foods, functional foods, and pharmaceuticals containing the biologically active substances. Example of such biologically active substances are liquid preparations containing lymphokines such as α-, β-, and γ-interferons, tumor necrosis factor-α (TNF-α), tumor necrosis factor-β (TNF-β), macrophage migration inhibitory factor, colony-stimulating factor, transfer factor, and interleukin 2; liquid preparations containing hormones such as insulin, growth hormone, prolactin, erythropoietin, and follicle-stimulating hormone; liquid biological preparations such as BCG vaccine, Japanese encephalitis vaccine, measles vaccine, live polio vaccine, small pox vaccine, tetanus toxoid, Trimeresurus antitoxin, and human immunoglobulin; liquid preparations containing antibiotics such as penicillin, erythromycin, chloramphenicol, tetracycline, streptomycin, and kanamycin sulfate; liquid preparations containing vitamins such as thiamin, riboflavin, L-ascorbic acid, cod liver oil, carotenoid, ergosterol, tocopherol; highly unsaturated fatty acids and their derivatives such as EPA, DHA and arachidonic acid; solution of enzymes such as lipase, esterase, urokinase, protease, β-amylase, isoamylase, glucanase, and lactase; extracts such as ginseng extract, "*suppon*"(soft-shelled turtle) extract, chlorella extract, aloe extract and propolis extract; healthy foods containing biologically active substances such as virus, living microorganisms paste of lactic acid bacteria and yeast, and royal jelly. By using cycloisomaltotetraose and the saccharide compositions comprising the same, the above biologically active substances can be arbitrary prepared in health foods, functional foods, and pharmaceuticals in a liquid, paste, or solid form, which have a satisfactorily-high stability and quality with less fear of losing or inactivating their effective ingredients and activities.

The methods for incorporating cycloisomaltotetraose or the saccharide composition comprising the same into the aforesaid compositions are those which can incorporate cycloisomaltotetraose and the saccharide compositions into the compositions before completion of their processing, and which can be appropriately selected from the following conventional methods; mixing, kneading, dissolving, melting, soaking, penetrating, dispersing, applying, coating, spraying, injecting, crystallizing, and solidifying. The amount of cycloisomaltotetraose or the saccharide compositions comprising the same to be preferably incorporated into the final compositions is usually in an amount of 0.1% or higher, desirably, 1% or higher.

The following experiments explain the present invention in detail.

### Experiment 1

### Products formed from dextran

### Experiment 1-1

### Preparation of crude enzyme

A liquid culture medium (pH 6.8) consisting of 15 g/L of "DEXTRAN T70", a commercially available dextran commercialized by Pharmacosmos A/s., Denmark, 1.0 g/L "POWDERY YEAST EXTRACT SH" a yeast extract commercialized by Nihon Pharmaceutical Co., Ltd., Tokyo, Japan, 5.0 g/L of "HIPOLYPEPTONE", a polypeptone commercialized by Nihon Pharmaceutical Co., Ltd., Tokyo, Japan, 1,0 g/L of dipotassium phosphate, 0.6 g/L of sodium phosphate dihydrate, 0.5 g/L of magnesium sulfate hepta-hydrate, 0.01 g/L of ferrous sulfate hepta-hydrate, 0.01 g/L of manganese sulfate penta-hydrate, 3.0 g/L of calcium carbonate and water was placed in twenty 500 mL-Erlenmeyer flasks in respective amounts of 100 mL, sterilized by autoclaving at 121°C for 20 min, and cooled. Successively, the culture medium was inoculated with a microorganism, strain D1110, and followed by cultivation under rotary-shaking conditions at 27°C and 240 rpm for 4 days. After completion of the cultivation, the culture supernatant was obtained by centrifuging the culture broth at 12,000 rpm for 10 minutes to remove cells. To about 0.9 L of the resulting culture supernatant, ammonium sulfate was admixed and dissolved to give 80%-saturation to precipitate proteins. The resulting precipitate was dissolved in 20 mM Tris-HCl buffer (pH 7.5), dialyzed against the same buffer at 4°C for 24 hours, and then centrifuged at 12,000 rpm for 10 min at 4°C. The resulting supernatant was collected as a crude enzyme solution.

### Experiment 1-2

### Action of the crude enzyme on dextran

One liter of 50 mM acetate buffer (pH 6.0) containing 1% (w/v) of "DEXTRAN T70", dextran commercialized by Pharmacosmos A/S, Denmark, and 0.006% (w/v) of hinokitiol as preservative was admixed with the crude enzyme solution obtained in Experiment 1-1, and reacted at 40°C for 20 hours. The reaction mixture was heated at 100°C for 10 min to inactivate the enzyme, concentrated using an evaporator, and dissolved in 100 mL of deionized water. Successively, the resulting solution was admixed with 200 mL of ethanol to precipitate high-molecular weight substances remaining in the reaction mixture such as dextran and proteins, and then the mixture was centrifuged and at 12,000 rpm for 20 min at 4°C, and the resulting supernatant was collected. The resulting supernatant was further subjected to membrane filtration using a membrane with a molecular weight cutoff of 10,000 to remove remaining high-molecular weight substances, and the resulting filtrate was collected as an enzymatic reaction product.

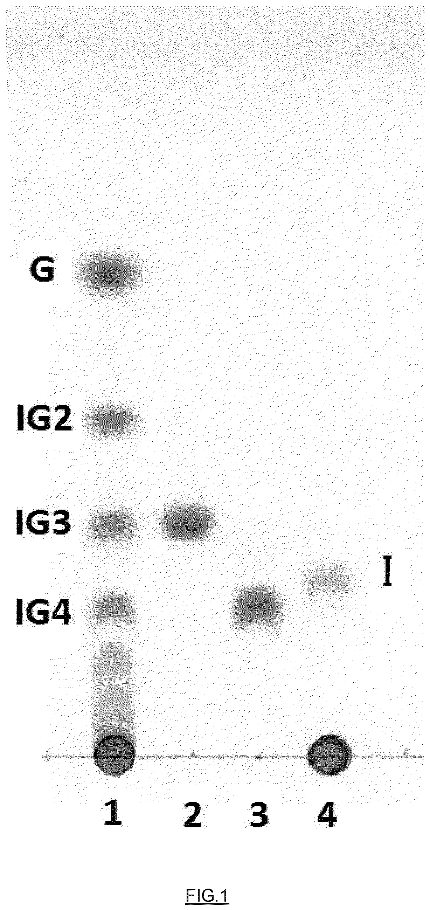

To examine saccharides in the resulting enzymatic reaction product, the product was subjected along with isomaltooligosaccharides markers (a preparation produced by Hayashibara Co., Ltd.), a standard product of isomaltotriose (a preparation produced by Hayashibara Co., Ltd.) and a standard product of isomaltotetraose (a preparation produced by Hayashibara Co., Ltd.) to thin layer chromatography (hereinafter, abbreviated as "TLC") to be developed twice using "KEISEL GEL 60" (aluminum plate, 10 × 20 cm, commercialized by Merck Co., Ltd.) as a thin-layer plate, and a solvent mixture (2-propanol: 1-butanol: water (12: 3: 4, v/v/v)) for development to separate the saccharides in the product. The saccharides separated on the TLC plate were detected by coloring by methanol-sulfuric acid method. The results are shown in FIG 1. As shown in FIG. 1, in comparison with the relative flow rate values (Rf values) of isomaltooligosaccharide markers (lane 1), the standard product of isomaltotriose (lane 2), and the standard product of isomaltotetraose (lane 3), developed at the same time, a spot of an unknown saccharide (symbol "I" in FIG. 1) in which the Rf value is lower than that of isomaltotriose having a glucose polymerization degree of 3 (symbol "IG3" in FIG. 1) and larger than that of isomaltotetraose having a glucose polymerization degree of 4 (symbol "IG4" in FIG. 1) was detected in lane 4 that the enzyme reaction products were separated. The unknown saccharide was named to "Unknown Saccharide I".

### Experiment 1-3

### Preparation of Unknown Saccharide I

In the preliminary test using a small amount of the reaction product, Unknown Saccharide I, since it was confirmed that Unknown Saccharide I is not hydrolyzed by α-glucosidase and glucoamylase, and not decomposed by alkaline treatment, subsequently, an experiment for hydrolyzing the dextran hydrolysates concomitant in Unknown Saccharide I to D-glucose. The enzymatic reaction product containing Unknown Saccharide I, obtained in Experiment 1-2, was adjusted to pH 5.0, admixed with 100 U/g-solid of "TRANSGLUCOSIDASE L Amano", α-glucosidase produced by Amano Enzyme Co., Ltd., and 1,000 U/g-solid of glucoamylase commercialized by Nagase ChemteX Co., Ltd., and then reacted at 50°C for 4 days. After the reaction, the reaction was stopped by heating the reaction mixture at about 100°C for 10 min. The saccharides in the resulting reaction mixture were analyzed by analytical high-performance liquid chromatography (hereinafter, abbreviated as "analytical HPLC"). As a result, D-glucose and Unknown Saccharide I were detected at elution times of 58.7 min and 49.4 min, respectively. The saccharide composition of the reaction mixture is 30.0% (w/w) of D-glucose, 65.5% (w/w) of Unknown Saccharide I, and 4.5% (w/w) of partial dextran hydrolysates containing isomaltooligosaccharides.

The analytical HPLC was carried out under the following conditions;
Column: "MCl gel CK04SS", produced by Mitsubishi Chemical Corporation, Tokyo, Japan; two columns were connected in series;
Eluent: water;
Column temperature: 80°C;
Flow rate: 0.4 mL/min;
Detector: "RID-10A", a differential refractometer produced by Shimadzu Corporation, Kyoto, Japan.

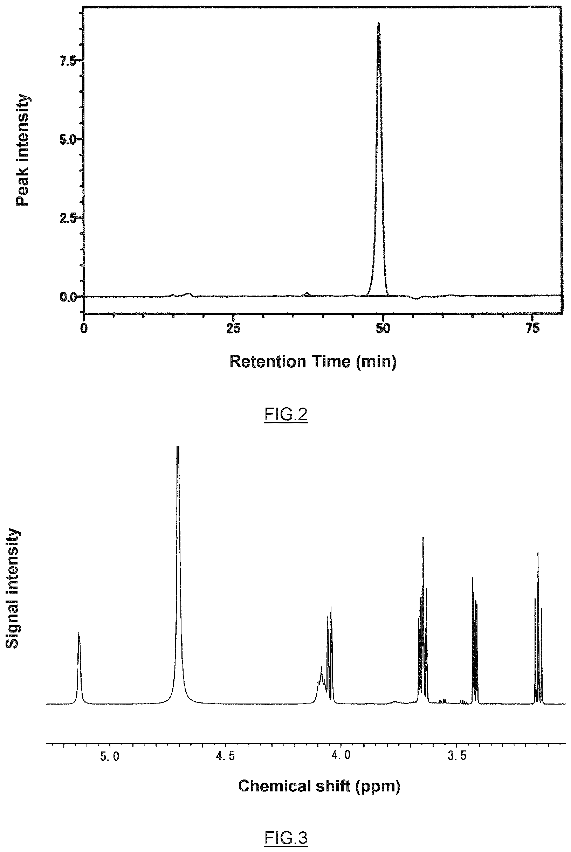

Subsequently, reducing saccharides (D-glucose and small amount of partial dextran hydrolysates) in the reaction mixture were decomposed by the steps of admixing sodium hydroxide with the reaction mixture obtained by α-glucosidase and glucoamylase treatment to adjust the pH to 12, and incubating at 98°C for one hour. After removing precipitates by filtration from the resulting solution, the resulting filtrate was admixed with "DIAION SK-1B", a cation exchanger resin produced by Mitsubishi Chemical Corporation, Tokyo, Japan, for neutralizing and adjusting the pH to 8, and then decolored by activated charcoal treatment. The resulting saccharide solution was further deionized using "DIAION SK-1B", "DIAION WA30", anion exchanger resin produced by Mitsubishi Chemical Corporation, Tokyo, Japan, and "IRA411" anion exchanger resin produced by Organo Corporation, Tokyo, Japan, filtrated by fine filtration, concentrated in an evaporator, and dried in vacuum. As a result, about 60 mg-dry solid of the purified preparation of Unknown Saccharide I was obtained. The saccharide composition of the resulting Unknown Saccharide I preparation was examined by analytical HPLC. As shown in HPLC chromatogram in FIG. 2, the purity of Unknown Saccharide I is 99.1% (w/w) and it was found to be a very high purity preparation.

### Experiment 2

### Structural analyses of Unknown Saccharide I

The purified preparation of Unknown Saccharide I, obtained in Experiment 1, was subjected to analyses by LC/MS and various NMR to determine the structure.

### Experiment 2-1

### Analysis by LC/MS

The mass of Unknown Saccharide I was measured by subjecting the purified preparation of Unknown Saccharide I obtained in Experiment 1 to "PROMINENCE", a LC/MS analyzer commercialized by Shimadzu Corporation, Kyoto, Japan, using the same LC conditions with HPLC in the above Experiment 1-3. In the HPLC, the peak of Unknown Saccharide I was eluted at the retention time of 49.5 min, and sodium-added molecular ion with a mass number (m/z) of 671 was significantly detected. Accordingly, it was revealed that the mass of Unknown Saccharide I is 648. The value of the mass suggested that Unknown Saccharide I is a cyclic gluco-tetrasaccharide having a structure that four D-glucose molecules are bound to give a cyclic form.

### Experiment 2-2

### Analyses by ¹H-NMR and ¹H-¹H COSY

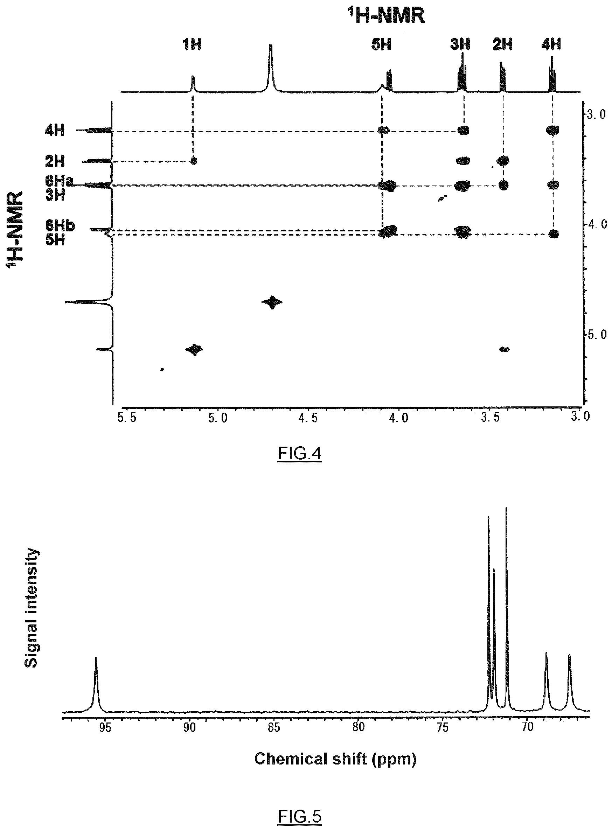

In order to determine the chemical structure of Unknown Saccharide I, the purified preparation of Unknown Saccharide I obtained in Experiment 1 was dissolved in heavy water (D₂O), and subjected to nuclear magnetic resonance (NMR) analyses, ¹H-NMR and ¹H-¹H-COSY, using "AVANCE II Spectrometer", a NMR apparatus commercialized by Bruker-Japan, Kanagawa, Japan. The ¹H-NMR spectrum and the ¹H-¹H COSY spectrum of Unknown Saccharide I are shown in FIG. 3 and FIG. 4, respectively.

As shown in FIG. 3, seven main peaks were observed in the ¹H-NMR spectrum of Unknown Saccharide I (Since heavy water was used as a solvent for NMR analysis, protons of OH groups were not detected in the spectrum). From the integration ratio of the peak, it was found that seven protons with different environments are present in the molecule of Unknown Saccharide I. The doublet peak observed at 5.13 ppm was assigned to the C-1 proton bound to anomeric carbon of D-glucose. Based on the ¹H-¹H COSY spectrum shown in FIG. 4, peaks were sequentially assigned based on the correlation with the C-1 proton. The results of the assignments of the seven proton peaks are shown in Table 1.

**Table 1**

| Chemical shift (ppm) | Coupling constant (Hz) | Splitting pattern | Assignment |
|---|---|---|---|
| 5.13 | 3.9 | d | 1H |
| 3.42 | 9.9, 3.8 | dd | 2H |
| 3.64 | 9.5 | t | 3H |
| 3.14 | 9.5 | t | 4H |
| 4.08 | 10.4 | t | 5H |
| 4.05 | 11.3, 2.6 | dd | 6Ha |
| 3.64 | 10.7 | t | 6Hb |

| | | | |
|---|---|---|---|
| d: Doublet dd: Double doublet t: Triplet 1H-6H: Protons bound to carbons at C1-C6 positions of D-glucose | | | |

From the values of the coupling constant and chemical shift of each proton peak shown in Table 1, it was found that Unknown Saccharide I has a glucopyranosyl structure, and the binding mode of D-glucoses of Unknown Saccharide I are α-anomer because the peak of C-1 proton bound to the anomeric carbon was detected at 5.13 ppm.

### Experiment 2-3

### Analyses by ¹³C-NMR, HSQC, and HMBC

Using the NMR apparatus used in Experiment 2-2, Unknown Saccharide I was subjected to ¹³C-NMR, HSQC (Heteronuclear Single-Quantum Correlation Spectroscopy) and HMBC (Heteronuclear Multiple Bond Correlation Spectroscopy) analyses. ¹³C-NMR, HSQC, and HMBC spectra of Unknown Saccharide I are shown in FIG. 5, FIG. 6, and FIG. 7, respectively.

As shown in FIG. 5, only six carbon peaks were observed in the ¹³C-NMR spectrum of Unknown Saccharide I. From the molecular mass obtained by LC/MS analysis in Experiment 2-1, it was suggested that Unknown Saccharide I is a cyclic gluco-tetrasaccharide having a structure of binding four D-glucose molecules to form cyclic structure. Since only six carbon peaks were observed, it was suggested that Unknown Saccharide I is a cyclic gluco-tetrasaccharide constructed by a single binding mode. Carbon observed at 95.522 ppm in the ¹³C-NMR spectrum was assigned to anomeric carbon.

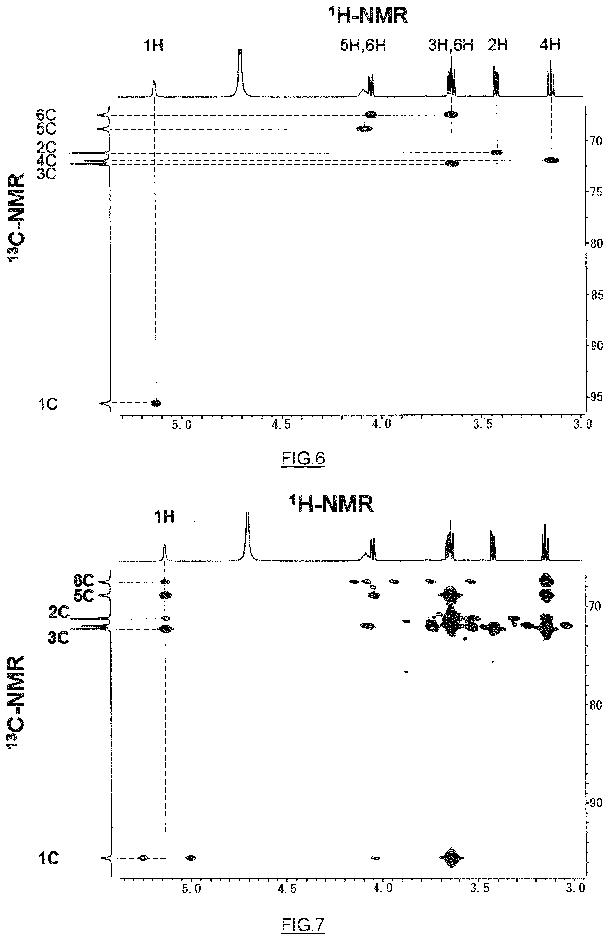

Based on the results of the assignment of the peaks detected in ¹H-NMR spectrum shown in Table 1, the carbon showing correlation with each proton in the HSQC spectrum shown in FIG. 6 were assigned and the results were shown in Table 2.

**Table 2**

| Chemical shift (ppm) | Assignment |
|---|---|
| 95.5 | 1C |
| 71.2 | 2C |
| 72.2 | 3C |
| 71.9 | 4C |
| 68.8 | 5C |
| 67.4 | 6C |

In the HMBC spectrum shown in FIG. 7, carbons showing a correlation with the C-1 proton were investigated. As the results, C-2, C-3, C-5, and C-6 carbons showed correlation with C-1 proton, and C-4 carbon does not show correlation. Since C-1 proton showed a correlation between C-6 carbon in addition to C-2, C-3, and C-5 carbons, it was revealed that in Unknown Saccharide I, glucosidic linkage is present between C-1 carbon and C-6 carbon, i.e., D-glucoses in Unknown Saccharide I are bound *via* 1,6-glucosidic linkages.

From the above results, it was revealed that Unknown Saccharide I is a cyclic glucotetrasaccharide having a structure of binding four D-glucose molecules to form a cyclic structure *via* α-1,6 glucoside linkages, i.e., cycloisomaltotetraose, as shown in FIG. 8. Since a saccharide having such a structure has not been known at all, cycloisomaltotetraose is a novel cyclic saccharide.

### Experiment 3

### Crystal of cycloisomaltotetraose

The preparation of the crystal of cycloisomaltotetraose was tried using a purified preparation of cycloisomaltotetraose (25.9 g, purity: 96.1% (w/w)) re-prepared by scaling-up the method in Experiment 1.

### Experiment 3-1

### Preparation of crystal

Crystals were precipitated by the step of suspending the purified preparation of cycloisomaltotetraose in deionized water; heating the resulting suspension to dissolve the saccharide; adjusting the saccharide concentration (Brix value) of the resulting cycloisomaltotetraose solution to 53.2 %; and cooling the solution to room temperature. A micrograph of the crystals is shown in FIG. 9. The precipitated crystals were collected by filtration and dried under a reduced pressure at 25°C, and then, 8.6 g of crystalline preparation of cycloisomaltotetraose was obtained. The purity of cycloisomaltotetraose of the crystals was determined by analytical HPLC described above and the value was high as 99.5% by weight, d.s.b.

### Experiment 3-2

### Analyses of crystalline cycloisomaltotetraose

The crystalline preparation of cycloisomaltotetraose obtained in Experiment 3-1 was subjected to a measurement of the moisture content analysis, thermal analysis, and powder X-ray diffraction analysis using the conventional methods.

### Moisture content

The moisture content of the crystalline preparation of cycloisomaltotetraose obtained in Experiment 3-1 was measured using "AVQ-2200A", a Karl-Fischer moisture measuring apparatus commercialized by Hiranuma Sangyo Co., Ltd., Ibaraki, Japan, and the value of 13.9% by weight was obtained. The theoretical value of the moisture content when assuming the crystalline cycloisomaltotetraose is pentahydrate crystal or hexahydrate crystal is 12.2% by weight or 14.3% by weight. Accordingly, the cycloisomaltotetraose crystal was considered to be either pentahydrate crystal or hexahydrate crystal.

### Thermal Analysis (DSC, TG/DTA)

About 5 mg of the crystalline preparation of cycloisomaltotetraose, obtained in Experiment 3-1, was weighed into an aluminum container and subjected to thermal analysis using "TG/DTA 6200", a differential heat weight simultaneous measuring apparatus commercialized by Hitachi High-Tech Science Corporation, Tokyo, Japan, with heating the container from 30°C to 300°C at a heating rate of 5°C/min under a nitrogen atmosphere. As a result, weight loss of 12.12% by weight was observed up to 116.5°C. The weight loss was considered to be due to desorbing of crystalline water. Considering the result with that for moisture content, combined with the above findings for moisture content, the crystalline cycloisomaltotetraose was considered to be pentahydrate crystal.

### Powder X-ray Diffraction Pattern

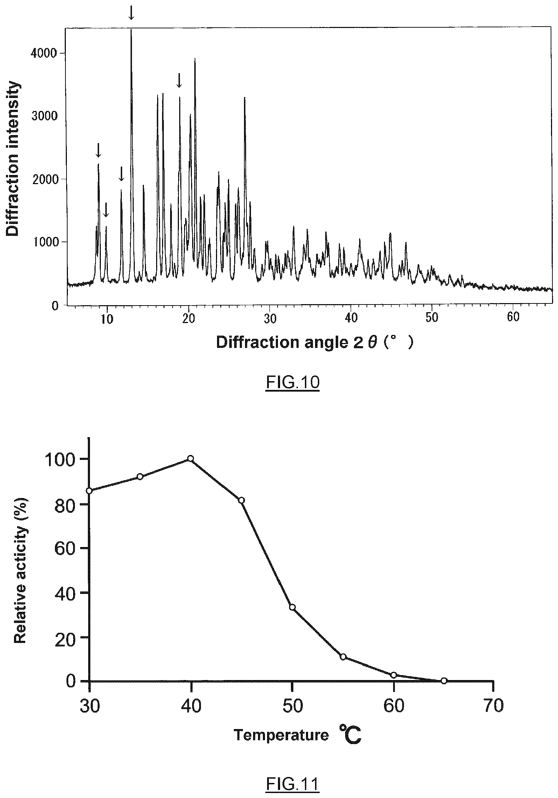

Powder X-ray diffraction analysis of crystalline cycloisomaltotetraose pentahydrate was carried out by placing the sample on a Si-reflection free sample holder and using "X' PERT PRO MPD", a powder X-ray (CuKα-ray) diffraction apparatus commercialized by Spectris Co., Ltd., Tokyo, Japan. The powder X-ray diffraction diagram of crystalline cycloisomaltotetraose pentahydrate is shown in FIG. 10. Characteristic diffraction peaks were observed at diffraction angles (2θ) of 9.0°, 9.8°, 11.8°, 13.1° and 19.1° (Symbols "↓" in FIG. 10).

### Experiment 4

### Identification of microorganisms capable of producing cycloisomaltotetraose-forming enzyme

In a screening from soil, two novel microorganisms, D1110 and D2006 were isolated, and D2006 showed a different colony form from D1110. By the microscopic observation, it was found that both microorganisms, D1110 and D2006, are rod shaped bacteria. In this experiment, species of the microorganisms, D1110 and D2006, were identified by determining the nucleotide sequences of those 16S rRNA (16S rDNA).

### Experiment 4-1

### Preparation of 16S rDNA from microorganisms, D1110 and D2006

A medium (pH 6.8), containing 1.5 g/L of "DEXTRAN T70", commercially available dextran commercialized by Pharmacosmos A/S, Denmark, 1.0 g/L of dipotassium phosphate, 1.0 g/L of magnesium sulfate heptahydrate, 1.0 g/L of sodium chloride, 2.0 g/L of ammonium sulfate, 0.01 g/L of ferrous sulfate heptahydrate, 0.01 g/L of manganese sulfate heptahydrate, 0.001 g/L of zinc sulfate heptahydrate, 20.0 g/L of gellan gum, and water, was sterilized by autoclaving at 121°C for 20 min, and then, the medium was poured into a shale and cooled to prepare gellan gum plate medium. Successively, microorganisms, D1110 and D2006, isolated from soil, were respectively inoculated into the plate medium, cultured at 27°C for 3 days for allowing to form single colonies.

Single colonies of D1110 and D2006 obtained above were respectively taken and suspended in 50 µL of "MIGHTYPREP REAGENT FOR DNA", a commercially available DNA simple extraction reagent commercialized by Takara Bio Inc., Shiga, Japan, and treated at 95°C for 10 min. After the treatment, the solution was centrifuged at 15,000 rpm for 2 min for collecting supernatant containing genomic DNA. Each genomic DNA of D1110 and D2006 was subjected to PCR using a sense primer, "8F", having a nucleotide sequence of SEQ ID NO: 1 and an antisense primer, "1512R", having a nucleotide sequence of SEQ ID NO: 2, and the resulting PCR-amplified product was subjected to agarose gel electrophoresis. Since a PCR-amplified product with a length of about 1.5 kbp was observed respectively, the products were collected by ethanol precipitation and used as 16S rDNA.

### Experiment 4-2

### Determination of the nucleotide sequence of 16S rDNA

Each nucleotide sequence of 16S rDNA from D1110 and D2006, obtained in Experiment 4-1, was determined by a conventional method, respectively. It was revealed that 16S rDNAs from D1110 and D2006 have nucleotide sequences of SEQ ID NO: 3 (1,447 bp) and SEQ ID NO: 4 (1,485 bp), respectively.

### Experiment 4-3

### Identification of microorganisms, D1110 and D2006, by homology search of those 16S rDNA

Based on the nucleotide sequence of 16S rDNA determined in Experiment 4-2, homology search was carried out for the databases RDP (The Ribosomal Database Project) and EzTaxon, using "GENETYX Ver. 13", a gene analysis software.

As the results of the homology search for 16S rDNA from D1110 obtained in Experiment 4-2, the genus, species, and strain, and the value of homology to their 16S rDNA nucleotide sequences of the top 5 among the top 20 entries are shown in Table 3. Further, the results of the homology search for 16S rDNA from D2006 obtained by the same method are also shown in Table 4.

**Table 3**

| Rank | Name | Strain | Accession No. | Homology(%) |
|---|---|---|---|---|
| 1 | *Agreia Pratensis* | VKM Ac-2510(T) | jgi.1118336 | 98.34 |
| 2 | *Agreia bicolorate* | VKM Ac-1804(T) | JYFC01000015 | 98.20 |
| 3 | *Subtercola boreus* | K300(T) | AF224722 | 97.28 |
| 4 | *Leifsonia antarctica* | SPC-20(T) | AM931710 | 97.23 |
| 5 | *Herbiconiux ginsengi* | CGMCC 4.3915(T) | jgi.1076294 | 97.10 |

**Table 4**

| Rank | Name | Strain | Accession No. | Homology(%) |
|---|---|---|---|---|
| 1 | *Microbacterium trichothecenolyticum* | DSM 8608 | JY JA0100006 | 99.17 |
| 2 | *Microbacterium hibisci* | THG-T2.14 | KX456190 | 98.46 |
| 3 | *Microbacterium jejuense* | THG-C31 | JX997974 | 98.38 |
| 4 | *Microbacterium yannicii* | G72 | FN547412 | 98.34 |
| 5 | *Microbacterium testaceum* | DSM 20166 | X77445 | 98.20 |

As shown in Table 3, the nucleotide sequence of 16S rDNA of D1110 showed the highest homology of 98.34% between that of *Agreia pratensis* VKM Ac-2510 (T). Generally, the classification of bacteria based on the 16S rDNA nucleotide sequence is recognized to be likely the same species if there is a homology of 99% or higher. Since there is no nucleotide sequence showing the homology of 99% or higher in the search, it could not lead to the identification of species. However, it was revealed that D1110 belongs to the genus *Agreia.* Based on the result, D1110 was identified to *Agreia* sp., and named to *Agreia* sp. D1110.

Further, as shown in Table 4, the nucleotide sequence of 16S rDNA of D2006 showed the highest homology of 99.17% between that of *Microbacterium trichothecenolyticum* DSM8608. Based on this result, the genus and species of D2006 was identified as *Microbacterium trichothecenolyticum,* and D2006 was named to *Microbacterium trichothecenolyticum* D2006.

Both *Agreia* sp. D1110 and *Microbacterium trichothecenolyticum* D2006 were deposited to the National Institute of Technology and Evaluation (NITE) Patent Microbiology Depositary Center (NPMD), respectively, and accepted on December 28, 2018 under the accession Nos. NITE BP-02815 and NITE BP-02816, respectively.

### Experiment 5

### Preparation of cycloisomaltotetraose-forming enzyme from Agreia sp. D1110 (NITE BP-02815)

The same liquid medium used in Experiment 1-1 was placed by 3mL in the test tube, sterilized by autoclaving at 121°C for 20 minutes, and cooled. Then, *Agreia* sp. D1110 (NITE BP-02815) was inoculated and cultured at 27°C, for 96 hours with shaking at 240 rpm to obtain a seed culture.

To 20 Erlenmeyer flasks with a 500 mL-volume, 100 mL each of the same liquid medium for the seed culture was dispensed, and the medium was sterilized by autoclaving at 121 °C for 20 min and cooled to 27°C. Then, 1 mL each of the seed culture obtained above was inoculated to each liquid culture medium and cultured by rotary-shaking at 27°C for 66 hours. After the cultivation, the culture broth was centrifuged at 12,000 rpm for 15 min to remove cells and about 1.8 L of culture supernatant was obtained. The culture supernatant was subjected to assay for cycloisomaltotetraose- forming enzyme activity, and it was revealed that cycloisomaltotetraose-forming enzyme activity of the culture supernatant was 0.112 unit/mL.

### Experiment 6

### Purification of enzyme from Agreia sp. D1110 (NITE BP-02815)

About 1.8 L of the culture supernatant (Total activity: 202 units) obtained in Experiment 5 was salted out by adding ammonium sulfate to give finally 80% saturation and allowing it to stand at 4°C for 24 hours. The resultant precipitates were collected by centrifuging at 12,000 rpm for 30 min, dissolved in 20 mM Tris-HCl buffer (pH 7.5), and dialyzed against the same buffer to obtain about 153 mL of a dialyzed enzyme solution. The dialyzed enzyme solution had 33.2 units of cycloisomaltotetraose-forming activity. The enzyme solution was then subjected to anion-exchange column chromatography using 24.1 mL of "DEAE-TOYOPEARL 650S" gel, an anion-exchange gel commercialized by Tosoh Corporation, Tokyo, Japan. Cycloisomaltotetraose-forming enzyme activity was adsorbed on "DEAE-TOYOPEARL 650S" gel equilibrated with 20 mM Tris-HCl buffer (pH 7.5) and eluted by 0 M-0.5 M NaCl linear gradient. The active fractions were eluted at about 0.25 M NaCl, collected and admixed with ammonium sulfate to give a final concentration of 1 M, and then centrifuged to remove precipitates. Successively, the enzyme solution was subjected to hydrophobic column chromatography using 11 mL of "PHENYL-TOYOPEARL 650M" gel, a hydrophobic gel commercialized by Tosoh Corporation, Tokyo, Japan. Cycloisomaltotetraose-forming enzyme activity was adsorbed on "PHENYL-TOYOPEARL 650M" gel equilibrated with 20 mM Tris-HCl buffer (pH 7.5) containing 1 M of ammonium sulfate and when eluted with a linear gradient decreasing from 1 M to 0 M of ammonium sulfate, cycloisomaltotetraose-forming enzyme activity was eluted at about 0.3 M of ammonium sulfate. The amount of total enzyme activity, total protein, specific activity and yield of cycloisomaltotetraose-forming enzyme in each purification step are shown in Table 5.

**Table 5**

| Purification step | Total activity (U) | Total protein (mg) | Specific activity (U/mg) | Yield (%) |
|---|---|---|---|---|
| Culture supernatant | 202.0 | 440 | 0.46 | 100 |
| Dialyzed solution after salting out with ammonium sulfate | 33.2 | 260 | 0.13 | 17 |
| Eluate form DEAE-TOYOPEARL 650S column chromatography | 21.8 | 25 | 0.88 | 11 |
| Eluate from PHENYL-TOYPEARL 650M column chromatography | 20.3 | 2.5 | 8.1 | 10 |

The purified enzyme preparation of cycloisomaltotetraose-forming enzyme, obtained by final hydrophobic chromatography using "PHENYL-TOYOPEARL 650M" gel, was assayed for purity on SDS-polyacrylamide gel electrophoresis using a 8-16% (w/v) gradient gel and detected on the gel as a single protein band, i.e., a high purity preparation.

### Experiment 7

### Properties of enzyme from Agreia sp. D1110 (NITE BP-02815)

### Experiment 7-1

### Molecular weight

The purified enzyme preparation of cycloisomaltotetraose-forming enzyme, obtained by the method in Experiment 6, was subjected to SDS-polyacrylamide gel electrophoresis (a 6 to 16% (w/v) gradient gel) and molecular weight of cycloisomaltotetraose-forming enzyme was measured comparing with those of "PRECISION PLUS UNCOLORED PROTEIN STANDARD", a molecular weight markers commercialized by Bio-Rad Japan, Tokyo, Japan, also subjected to the electrophoresis at the same time. It was revealed that cycloisomaltotetraose-forming enzyme has a molecular weight of 86,000 ± 10,000 daltons.

### Experiment 7-2

### Optimum temperature and optimum pH for the enzyme reaction

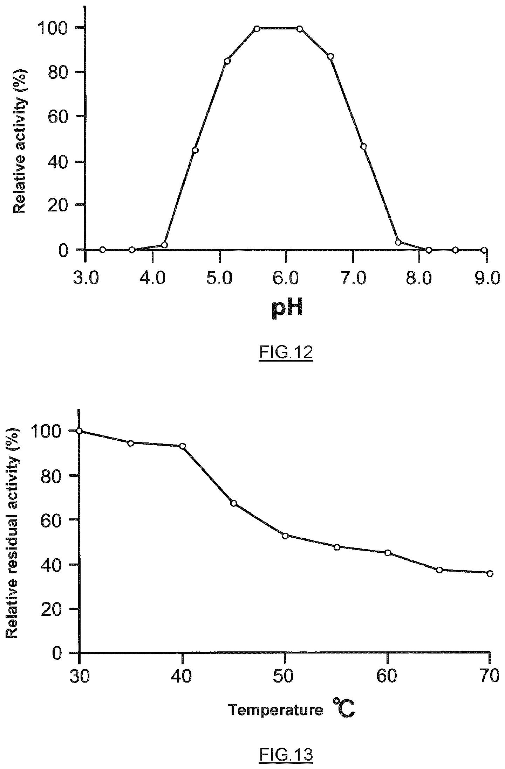

Effects of temperature and pH on the enzyme activity were investigated using the purified enzyme preparation of cycloisomaltotetraose-forming enzyme, obtained by the method in Experiment 6, by varying temperature and pH at the assay of the enzyme. The results are shown in FIG. 11 (Optimum temperature) and FIG. 12 (Optimum pH), respectively. It was revealed that the optimum temperature of cycloisomaltotetraose-forming enzyme is 35 to 40°C when reacted at pH 6.0 for 30 min and the optimum pH is 5.5 to 6.0 when reacted at 40°C for 30 min.

### Experiment 7-3

### Thermal and pH stabilities of the enzyme

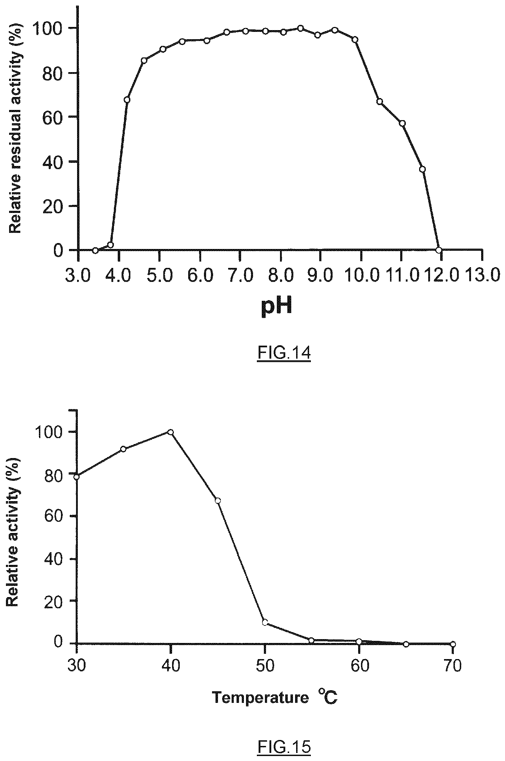

Thermal stability and pH stability of the enzyme were investigated using the purified enzyme preparation of cycloisomaltotetraose-forming enzyme, obtained by the method in Experiment 6. Thermal stability of the enzyme was determined by the steps of incubating an enzyme solution (20 mM acetate buffer, pH 6.0) under various temperatures for 30 min, cooling in water, and measuring the residual enzyme activity. pH Stability of the enzyme was determined by the steps of incubating enzyme solution in 100 mM buffer at various pHs and at 4°C for 16 hours, adjusting the pH to 6.0, and measuring the residual enzyme activity. The results are shown in FIG. 13 (Thermal stability) and in FIG. 14 (pH Stability), respectively. As is evident from the results in FIG. 13, cycloisomaltotetraose-forming enzyme is stable up to 40°C. As is evident from the results in FIG. 14, it was revealed that cycloisomaltotetraose-forming enzyme is stable in the range of pH 5.0 to 10.0.

### Experiment 7-4

### N-Terminal (NH₂-Terminal) amino acid sequence

The N-terminal amino acid sequence of the enzyme from D1110 was determined using the purified enzyme preparation of cycloisomaltotetraose-forming enzyme, obtained by the method in Experiment 6, by using "Model PPSQ-31A", a protein sequencer commercialized by Shimadzu Corporation, Kyoto, Japan. As a result, it was revealed that the enzyme has the N-terminal amino acid sequence of SEQ ID NO: 5, i.e., Ala-Thr-Val-Gly-Asp-Ala-Trp-Thr-Asp-Lys-Ala-Arg-Tyr-Asp-Pro.

### Experiment 8

### Preparation of enzyme from Microbacterium trichothecenolyticum D2006 (NITE BP-02816)

The same liquid medium used in Experiment 1-1 was placed by 3mL in the test tube, sterilized by autoclaving at 121°C for 20 min, and cooled. Then, *Microbacterium trichothecenolyticum* D2006 (NITE BP-02816) was inoculated and cultured at 27°C for 96 hours with shaking at 240 rpm to obtain a seed culture.

To 15 Erlenmeyer flasks with a 500 mL-volume, 100 mL each of the same liquid medium for the seed culture was dispensed, and the medium was sterilized by autoclaving at 121°C for 20 min and cooled to 27°C. Then, 1 mL each of the seed culture obtained above was inoculated to each liquid culture medium and cultured by rotary-shaking at 27°C for 66 hours. After the cultivation, the culture was centrifuged at 12,000 rpm for 15 min to remove cells and about 1.4 L of culture supernatant was obtained. The culture supernatant was subjected to assay for cycloisomaltotetraose-forming enzyme activity, and it was revealed that cycloisomaltotetraose-forming enzyme activity of the culture supernatant was 0.069 unit/mL.

### Experiment 9

### Purification of cycloisomaltotetraose-forming enzyme from Microbacterium trichothecenolyticum D2006 (NITE BP-02816)

About 1.4 L of the culture supernatant (Total activity: 96.6 units) obtained in Experiment 8 was salted out by adding ammonium sulfate to give finally 80% saturation and allowing it to stand at 4°C for 16 hours. The resultant precipitates were collected by centrifuging at 12,000 rpm for 30 min, dissolved in 20 mM Tris-HCl buffer (pH 7.5) containing 1 mM calcium chloride, and dialyzed against the same buffer to obtain about 110 mL of a dialyzed enzyme solution. The dialyzed enzyme solution had 140.7 units of cycloisomaltotetraose-forming activity. The enzyme solution was subjected to anion-exchange column chromatography using 20 mL of "DEAE-TOYOPEARL 650S" gel, an anion-exchange gel commercialized by Tosoh Corporation, Tokyo, Japan. Cycloisomaltotetraose-forming enzyme activity was adsorbed on "DEAE-TOYOPEARL 650S" gel equilibrated with 20 mM Tris-HCl buffer (pH 7.5) containing 1 mM calcium chloride and eluted with a linear gradient from 0 M to 0.5 M of NaCl. The active fractions eluted at about 0.2 M NaCl was collected and dialyzed against 20 mM Tris-HCI buffer (pH7.5) containing 1 mM calcium chloride. Successively, the dialyzed enzyme solution was subjected to anion-exchange column chromatography using 1.0 mL of "Mono Q 5/50GL" gel, a gel commercialized by GE Healthcare Japan, Tokyo, Japan. Cycloisomaltotetraose-forming enzyme activity was adsorbed on "Mono Q 5/50GL" gel equilibrated with 20 mM Tris-HCl buffer (pH 7.5) containing 1 mM calcium chloride and when eluted with a linear gradient from 0 M to 0.5 M of NaCl, cycloisomaltotetraose-forming enzyme activity was eluted at about 0.3 M NaCl. The amount of total enzyme activity, total protein, specific activity and yield of cycloisomaltotetraose-forming enzyme in each purification step are shown in Table 6.

**Table 6**

| Purification step | Total activity (U) | Total protein (mg) | Specific activity (U/mg) | Yield (%) |
|---|---|---|---|---|
| Culture supernatant | 96.6 | 387.8 | 0.25 | 100 |
| Dialyzed solution after salting out with ammonium sulfate | 140.7 | 143.1 | 0.98 | 146 |
| Eluate form DEAE-TOYOPEARL 650S column chromatography | 50.0 | 31.9 | 1.57 | 51.3 |
| Eluate from PHENYL-TOYPEARL 650M column chromatography | 10.5 | 2.95 | 3.55 | 10.9 |

The purified enzyme preparation of cycloisomaltotetraose-forming enzyme, obtained by hydrophobic chromatography using "Mono Q 5/50GL" gel, was assayed for purity on SDS-polyacrylamide gel electrophoresis (a 8-16% (w/v) gradient gel) and detected on the gel as a single protein band, i.e., a high purity preparation.

### Experiment 10

### Properties of cycloisomaltotetraose-forming enzyme from Microbacterium trichothecenolyticum D2006 (NITE BP-02816)

### Experiment 10-1

### Molecular weight

The purified enzyme preparation of cycloisomaltotetraose-forming enzyme, obtained by the method in Experiment 9, was subjected to SDS-polyacrylamide gel electrophoresis (a 6-16% (w/v) gradient gel) and molecular weight of cycloisomaltotetraose-forming enzyme was measured comparing with "PRECISION PLUS UNCOLORED PROTEIN STANDARD", a molecular weight markers commercialized by Bio-Rad Japan, Tokyo, Japan, also subjected to the electrophoresis at the same time. It was revealed that cycloisomaltotetraose-forming enzyme has a molecular weight of 86,000 ± 10,000 daltons.

### Experiment 10-2

### Optimum temperature and optimum pH for the enzyme reaction

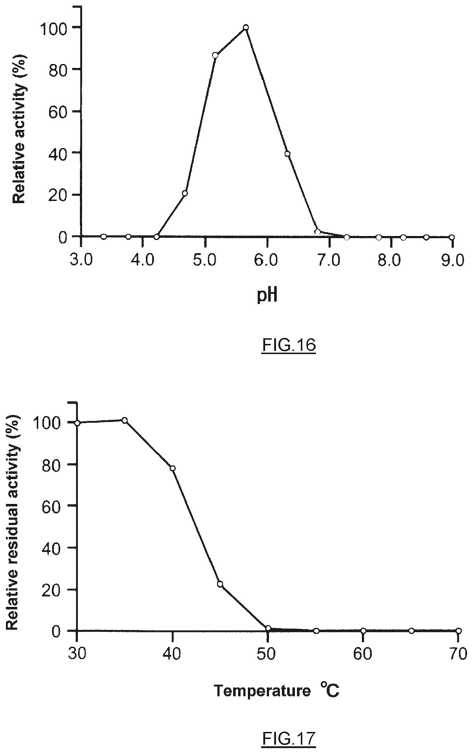

Effects of temperature and pH on the enzyme activity were investigated using the purified enzyme preparation of cycloisomaltotetraose-forming enzyme, obtained by the method in Experiment 9, by varying temperature and pH at the assay of the enzyme. The results are shown in FIG. 15 (Optimum temperature) and FIG. 16 (Optimum pH), respectively. It was revealed that the optimum temperature of cycloisomaltotetraose-forming enzyme is 40°C when reacted at pH 6.0 for 30 min and the optimum pH is around 5.5 when reacted at 40°C for 30 min.

### Experiment 10-3

### Thermal and pH stabilities of the enzyme

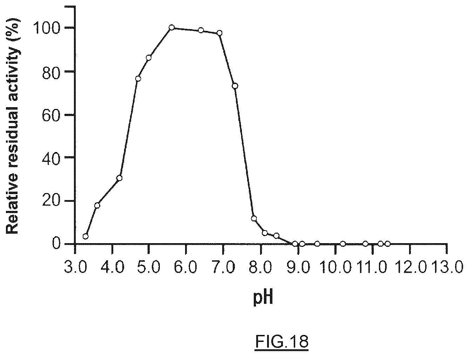

Thermal stability and pH stability of the enzyme were investigated using the purified enzyme preparation of cycloisomaltotetraose-forming enzyme, obtained by the method in Experiment 9. Thermal stability of the enzyme was determined by the steps of incubating an enzyme solution (20 mM acetate buffer, pH 6.0) under various temperatures for 30 min, cooling in water, and measuring the residual enzyme activity. pH Stability of the enzyme was determined by the steps of incubating enzyme solution in 100 mM buffer at various pHs, and at 4°C for 18 hours, adjusting the pH to 6.0, and measuring the residual enzyme activity. The results are shown in FIG. 17 (Thermal stability) and in FIG. 18 (pH Stability), respectively. As is evident from the results in FIG. 17, cycloisomaltotetraose-forming enzyme is stable up to 35°C. As is evident from the results in FIG. 18, it was revealed that cycloisomaltotetraose-forming enzyme is stable in the range of pH 5.0 to 7.0.

### Experiment 10-4

### N-Terminal amino acid sequence

The N-terminal amino acid sequence of the enzyme from D2006 was determined using the purified enzyme preparation of cycloisomaltotetraose-forming enzyme, obtained by the method in Experiment 9, by using "Model PPSQ-31A", a protein sequencer commercialized by Shimadzu Corporation, Kyoto, Japan. As a result, it was revealed that the enzyme has the N-terminal amino acid sequence of SEQ ID NO: 6, i.e., Ala-Asp-Leu-Gly-Asp-Ala-Trp-Thr- Asp.

### Experiment 11

### Cloning of a DNA encoding cycloisomaltotetraose-forming enzyme and preparation of a recombinant DNA comprising the DNA and a transformant

DNAs encoding cycloisomaltotetraose-forming enzyme were respectively cloned from *Agreia* sp. D1110 (NITE BP-02815) and *Microbacterium trichothecenolyticum* D2006 (NITE BP-02816), and self-replicable recombinant DNAs containing the DNA were constructed. Successively, the nucleotide sequences of the DNAs encoding the enzyme were determined and transformants were also prepared.

### Experiment 11-1

### Preparation of genomic DNAs

Cells of *Agreia* sp. D1110 (NITE BP-02815) or *Microbacterium trichothecenolyticum* D2006 (NITE BP-02816), subcultured on agar medium plate, was taken in an amount of one loop from the plate, inoculated to 3 mL of the liquid medium used in Experiment 1 in a test tube, and cultured under shaking conditions at 27°C and 240 rpm for 5 days. After completion of the cultivation, cells were collected by centrifuging the culture broth, and genomic DNA was prepared by a conventional method using "DNeasy Blood & Tissue Kit", a commercially available whole DNA purification kit, commercialized by QIAGEN N. V., Netherland.

### Experiment 11-2

### Determination of the nucleotide sequence of whole genome using a next-generation sequencer

Each genomic DNA from respective microorganism, obtained in Experiment 11-1, was enzymatically fragmented using "Nextera XT DNA Library Preparation Kit", a commercially available kit commercialized by Illumina K. K., Tokyo, Japan, and the resulting DNA fragments were treated to have brant ends, added the adaptor sequences, and made into a library. After amplifying the library by PCR, the amplified library was purified by using "AMPure XP", a commercially available DNA purification kit commercialized by Beckman Coulter Inc., USA. Then, the nucleotide sequences of the DNA fragments in the library were determined by using "MiSeq", a next-generation sequencer commercialized by Illumina K. K., Tokyo, Japan, and the nucleotide sequence of the whole genomic DNA was obtained by integrating the above nucleotide sequences (contig sequences) using a computer.

Successively, the nucleotide sequence of the whole genomic DNA was analyzed by using "Glimmer", a gene region-predicting software and open reading frames (ORF: deduced gene region) being estimated to encode proteins were predicted. As the results, it was found that the whole genomic DNA of *Agreia* sp. D1110 (NITE BP-02815) has 9,139 ORFs and that of *Microbacterium trichothecenolyticum* D2006 (NITE BP-02816) has 11,068 ORFs.

### Experiment 11-3

### Identification of an ORF encoding cycloisomaltotetraose-forming enzyme

An ORF encoding an amino acid sequence that matches with the N-terminal amino acid sequence of cycloisomaltotetraose-forming enzyme, determined in Experiment 3-2, (SEQ ID NO: 5) was searched from 9,139 ORFs of the whole genomic DNA from *Agreia* sp. D1110 (NITE BP-02815), detected in whole genome analysis in Experiment 11-2. As the result, it was found that an amino acid sequence identical to the N-terminal amino acid sequence was encoded in ORF9038. From the result, it was found that the nucleotide sequence of ORF9038, i.e., the nucleotide sequence of SEQ ID NO: 9 is that of the structural gene DNA of the cycloisomaltotetraose-forming enzyme from *Agreia* sp. D1110 (NITE BP-02815), and also found that the cycloisomaltotetraose-forming enzyme has an amino acid sequence that N-terminal amino acid sequence consisting of 31 amino acids deduced to secretion signal sequence was deleted from the amino acid sequence described together with the nucleotide sequence of SEQ ID NO: 9, i.e., amino acid sequence of SEQ ID NO: 7.

An ORF encoding an amino acid sequence that matches with the N-terminal amino acid sequence of cycloisomaltotetraose-forming enzyme determined in Experiment 10-4, (SEQ ID NO: 6) was searched from 11,068 ORFs of the whole genomic DNA from *Microbacterium trichothecenolyticum* D2006 (NITE BP-02816), detected in whole genome analysis in Experiment 11-2. As the result, it was found that an amino acid sequence identical to the N-terminal amino acid sequence was encoded in ORF5328. From the result, it was found that the nucleotide sequence of ORF5328, i.e., the nucleotide sequence of SEQ ID NO: 10 is that of the structural gene DNA of the cycloisomaltotetraose-forming enzyme from *Microbacterium trichothecenolyticum* D2006 (NITE BP-02816), and also found that the cycloisomaltotetraose-forming enzyme has an amino acid sequence that N-terminal amino acid sequence consisting of 32 amino acids deduced to secretion signal sequence was deleted from the amino acid sequence described together with the nucleotide sequence of SEQ ID NO: 10, i.e., amino acid sequence of SEQ ID NO: 8.

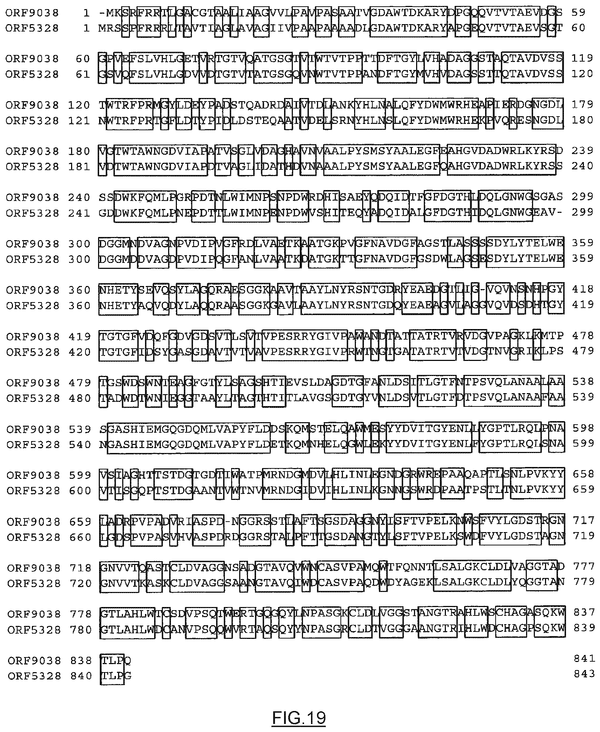

The molecular weight of the cycloisomaltotetraose-forming enzyme from *Agreia* sp. D1110 (NITE BP-02815) having the amino acid sequence of SEQ ID NO: 7 was calculated to be 86,350, and that of the enzyme from *Microbacterium trichothecenolyticum* D2006 (NITE BP-02816) having the amino acid sequence of SEQ ID NO: 8 was calculated to be 86,658. These values were well matched with the molecular weights of 86,000 ± 10,000 obtained by SDS-PAGE in Experiments 3-1 and 10-1, respectively. The homology (sequence identity) between the amino acid sequence encoded by the nucleotide sequence of SEQ ID NO: 9, that of the enzyme gene from *Agreia* sp. D1110 (NITE BP-02815) and the amino acid sequence encoded by the nucleotide sequence of SEQ ID NO: 10, that of the enzyme gene from *Microbacterium tichothecenolyticum* D2006 (NITE BP-02816) was investigated by using "GENETYX Ver. 13", a commercially available genetic information processing software commercialized by Genetyx Corporation, Tokyo, Japan, and it was calculated to be 71% (see FIG. 19).

### Experiment 12

### Preparation of a recombinant DNA for expression, pRSET A-ORF9038 and production of a recombinant cycloisomaltotetraose-forming enzyme by its transformant, E9038

The gene encoding cycloisomaltotetraose-forming enzyme in ORF9038 was amplified from genomic DNA from *Agreia* sp. D1110 (NITE BP-02815) by conventional PCR method and inserted to "pRSET A", an expression vector commercialized by Thermo Fisher Scientific K. K., Tokyo, Japan, and the expression of a recombinant cycloisomaltotetraose-forming enzyme in E. *coli* was investigated.

### Experiment 12-1

### Preparation of a recombinant DNA for expression, pRSET A-ORF9038 and a transformant, E9038

In order to insert the gene encoding cycloisomaltotetraose-forming enzyme from *Agreia* sp. D1110 (NITE BP-02815) to "pRSET A", an expression vector, an amplified DNA fragment was obtained by PCR against the whole genomic DNA of *Agreia* sp. D1110 (NITE BP-02815) using a sense primer having a nucleotide sequence of SEQ ID NO: 11 and an anti-sense primer having a nucleotide sequence of SEQ ID NO: 12 in combination. The PCR amplified DNA was designed to have nucleotide sequences of 15 bases homologous to pRSET A at both 5'- and 3'-ends for inserting it into the vector, pRSET A, by In-fusion method, to place the DNA encoding the amino acid sequence of the mature cycloisomaltotetraose-forming enzyme, i.e., amino acid sequence of SEQ ID NO: 7, just after initiation codon ATG, and to place termination codon TAA TAG just after the DNA. T7 promoter, ribosome-binding site (RBS) and initiation codon ATG were used those present on pRSET A.

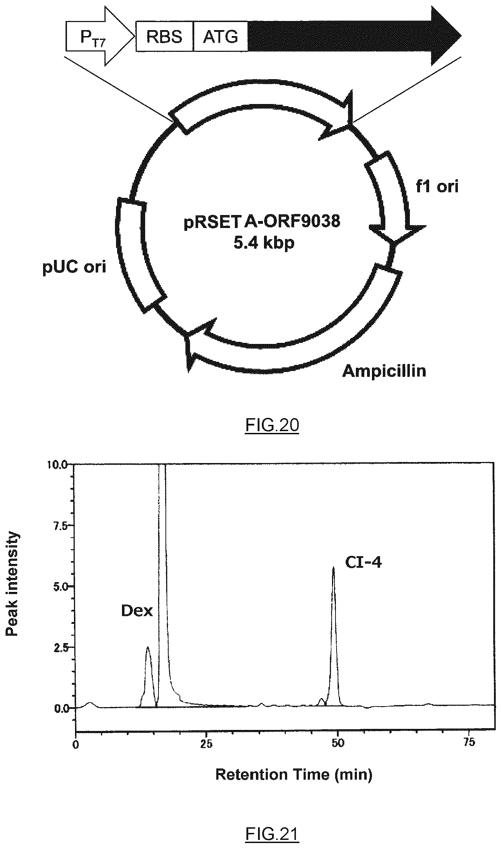

A recombinant DNA, obtained by inserting the above amplified DNA into the vector pRSET A by In-fusion method, was named to "pSET A-ORF9038". pRSET A-ORF-9038 is shown in FIG. 20. Then, *E. coli* HST-08, commercialized by Takara Bio Inc., Shiga, Japan, was transformed by using pSET A-ORF9038, and pRSET A-ORF9038 was isolated from the resulting transformant. Further, a host E. *coli* BL21 (DE3) for expression, commercialized by Merk Japan, was transformed by using the isolated pRSET A-ORF9038 to obtain a transformant, "E9038".

### Experiment 12-2

### Production of a recombinant cycloisomaltotetraose-forming enzyme by the transformant, E9038

A liquid TB medium (pH 6.8) consisting of 47 g/L of "TERRIFIC BROTH" commercialized by Thermo Fisher Scientific K. K., Tokyo, Japan, 4 g/L of glycerol and water was placed in a glass tube in respective amount of 5 mL, sterilized by autoclaving at 121°C for 20 min, and cooled. Then, ampicillin was aseptically admixed into the liquid medium to give a final concentration of 100 µg/mL. The transformant, E9038, obtained by the method in Experiment 12-1, was inoculated into the above liquid medium, and cultured at 37°C for 8 hours under a shaking condition, and then admixed with isopropyl-1-thio-β-D-galactopyranoside (IPTG) to give a final concentration of 100 µM for inducing the expression of a gene encoding cycloisomaltotetraose-forming enzyme, and the cultivation was further continued for 16 hours. Cells were collected from the culture broth by centrifugation. According to the conventional methods, cells were subjected to lysozyme treatment, freeze-thaw treatment, and ultrasonic disruption to obtain cell extract. The ultrasonic disruption was carried out by suspending cells in 20 mM Tris-HCl buffer (pH 7.5) and disrupting cells in suspension in an ice bath using a ultrasonic homogenizer, "Model UH-600", commercialized by MST Corporation, Aichi, Japan, and the resulting cell extract was centrifuged to obtain supernatant as a cell-free extract.

The cell-free extract thus obtained was used as a crude enzyme solution of the recombinant cycloisomaltotetraose-forming enzyme, and 0.3 mL of the crude enzyme solution was admixed with 0.3 mL of 200 mM acetate buffer (pH 6.0) containing 2.0% (w/v) of "DEXTRAN T70", a dextran commercialized by Pharmacosmos A/s., Denmark. After incubating the resulting mixture at 40°C for overnight, the mixture was heated at 100°C for 10 min to stop the reaction. The reaction mixture thus obtained was subjected to the analytical HPLC used in Experiment 1. The HPLC chromatogram is in FIG. 21. As a control, the E. *coli* BL21 (DE3) introduced with the expression vector, pRSET A, was cultured under the same conditions as in the case of transformants described above, to prepare a cell-free extract from the culture, and then the cycloisomaltotetraose-forming activity was examined by the same manner.

As shown in FIG. 21, in the HPLC chromatogram of the reaction mixture, a peak of dextran (symbol "Dex" in FIG. 21) used as substrate was eluted at the retention time of about 17 min, and a peak of cycloisomaltotetraose (symbol "CI-4" in FIG. 21) was detected at the retention time of about 49.4 min. From the result, it was qualitatively confirmed that cycloisomaltotetraose- forming enzyme gene is expressed in cells of the host E. *coli* and cycloisomaltotetraose-forming enzyme is produced. Although a concrete chromatogram was omitted, the formation of cycloisomaltotetraose was not detected in the case of using the cell-free extract from the control host *E. coli,* and the enzyme activity could not be detected at all.

Although the details were omitted, also in the case of the enzyme from *Microbacterium trichothecenolyticum* D2006 (NITE BP-02816), the recombinant DNA and transformant were prepared and the recombinant enzyme was expressed in cells of host E. *coli* in the same manner of the above enzyme from *Agreia* sp. D1110 (NITE BP-02815), and as the result, the production of cycloisomaltotetraose-forming enzyme was detected.

### Experiment 13

### Action of cycloisomaltotetraose-forming enzyme on isomaltopentaose

As a part of examining the substrate specificity of cycloisomaltotetraose-forming enzyme, the action of the enzyme on isomaltopentaose was investigated. To 0.5 mL of 50 mM acetate buffer (pH 6.0) containing 1% (w/v) of isomaltopentaose (prepared in Hayashibara Co., Ltd., Okayama, Japan, purity: 98.1% by weight) and 0.006% (w/v) hinokitiol as a preservative, 2.0 units/g-isomaltopentaose of a purified cycloisomaltotetraose-forming enzyme from *Agreia* sp. D1110 (NITE BP-02815), obtained by the method in Experiment 6, was added, and the resulting mixture was incubated at 40°C for 24 hours and then heated at 100°C for 10 min to inactivate the enzyme. To examine the reaction products in the reaction mixture, saccharides were separated by TLC using "KIESELGEL 60", a TLC aluminum plate (10 x 20 cm) and a solvent (acetonitrile/water/ethyl acetate/1-propanol, volume ratio of 85:70:20:50) and two-times ascending method. The separated saccharides on the plate were detected by visualizing the spots with sulfate-methanol method, and the reaction products formed from isomaltopentaose were investigated.

As the result, it was found that cycloisomaltotetraose-forming enzyme acts on isomaltopentaose and produces cycloisomaltotetraose with isomaltotetraose, also slightly isomaltotriose, isomaltose, glucose and high molecular glucans. From this result, it was considered that the enzyme acts on α-1,6 glucans with a glucose polymerization degree of at least 5 or higher.

### Experiment 14

### Substrate specificity of cycloisomaltotetraose-forming enzyme

Substrate specificity of cycloisomaltotetraose-forming enzyme was investigated using various saccharides as substrates. Aqueous solutions containing D-glucose, maltose, isomaltose, panose, isomaltotriose, isomaltotetraose, isomaltopentaose, isomaltohexaose, isomaltoheptaose, dextran T10 (average molecular weight: 10,000, commercialized by Pharmacosmos A/s., Denmark), dextran T70 (average molecular weight: 70,000, commercialized by Pharmacosmos A/s., Denmark), or dextran T2000 (average molecular weight: 2,000,000, commercialized by Pharmacosmos A/s., Denmark) were prepared as substrate solutions. To each substrate solution, acetate buffer and calcium chloride were added to give final concentrations of 50 mM and 1 mM, respectively, and then resulting solutions were admixed with 20 units/g-solid of cycloisomaltotetraose-forming enzyme from *Agreia* sp. D1110 (NITE BP-02815), obtained by the method in Experiment 6, adjusted the concentration to 1% (w/v) and incubated at pH 6.0, 40°C for 24 hours. To examine the saccharides in each reaction mixture before and after the reaction, saccharides were separated by silica gel thin-layer chromatography (hereinafter, abbreviated as "TLC") using "KIESELGEL 60F₂₅₄", a TLC aluminum plate (10 x 20 cm) and a solvent (2-propanol/1-butanol/water, volume ratio of 12:3:4) with two-times ascending method. The separated saccharides on the plate were detected by visualizing the spots with sulfate-methanol method, and the degree of forming cycloisomaltotetraose from each substrate and the products other than cycloisomaltotetraose were investigated. The results are shown in Table 7.

**Table 7**

| Substrate | Formation of cycloisomaltotetraose |
|---|---|
| D-Glucose | - |
| Maltose | - |
| Isomaltose | - |
| Panose | - |
| Isomaltotriose | - |
| Isomaltotetraose | + |
| Isomaltopentaose | ++ |
| Isomaltohexaose | +++ |
| Isomaltoheptaose | +++ |
| Dextran (Average molecular weight: 10,000) | +++ |
| Dextran (Average molecular weight: 70,000) | +++ |
| Dextran (Average molecular weight: 2,000,000) | +++ |

| | |
|---|---|
| ... - : Not formed + : Slightly formed ++ : Formed +++ : Significantly formed | |

As shown in Table 7, among the tested saccharides, cycloisomaltotetraose-forming enzyme did not act on monosaccharide, disaccharides, and trisaccharides, such as D-glucose, maltose, isomaltose, panose, and isomaltotriose. While, the enzyme acted on isomaltotetraose, isomaltopentaose, isomaltohexaose, isomaltoheptaose, and dextrans and formed cycloisomaltotetraose. From the results, it was found that the enzyme acts on α-1,6 glucans with a glucose polymerization degree of 4 or higher and forms cycloisomaltotetraose. The enzyme formed isomaltotetraose by hydrolyzing the formed cycloisomaltotetraose. Further, it was found that the enzyme acts on α-1,6 glucans with a glucose polymerization degree of 4 or higher and catalyzes disproportionation reaction of forming a series of isomaltooligosaccharides with a glucose polymerization degree of 2 or higher. In addition, the enzyme formed isomaltohexaose when allowed to act on cycloisomaltotetraose in the presence of isomaltose as an acceptor. From the result, it was found that the enzyme catalyzes a coupling reaction of hydrolyzing cycloisomaltotetraose and transferring the formed isomaltotetraose to isomaltose. In the same manner, substrate specificity of cycloisomaltotetraose-forming enzyme from *Microbacterium trichothecenolyticum* D2006 (NITE BP-02816) was also investigated, and it was found that the enzyme also acts on α-1,6 glucans with a glucose polymerization degree of 4 or higher and forms cycloisomaltotetraose as in the case of the enzyme from *Agreia* sp. D1110 (NITE BP-02815). Further, the enzyme was same with that from D1110 in the points of catalyzing hydrolysis of cycloisomaltotetraose, disproportionation of α-1,6 glucans, and coupling reaction. Overall, the substrate specificity of the enzyme was equivalent to the enzyme from D1110.

### Experiment 15

### Digestivity test of cycloisomaltotetraose using small intestinal mucosal enzyme

The crystalline cycloisomaltotetraose preparation with a purity of 99.5% by weight, obtained in Experiment 3-1, was dissolved in 50 mM maleic acid buffer (pH 6.0) to give a concentration of 1% by weight to make into a substrate solution. To the substrate solution, 50 units/g-cycloisomaltotetraose, as maltose-hydrolyzing activity, of small intestine mucosal enzyme (α-glucosidase) prepared from rat small intestine acetone powder, commercialized by Sigma-Aldrich Co., was added, and the resulting mixture was incubated at 37°C for 24 hours and then heated at 100°C for 10 min to inactivate the enzyme. The amounts of cycloisomaltotetraose in the solution before and after the reaction were determined by HPLC, and the residual rate (%) of cycloisomaltotetraose after the enzymatic reaction, i.e., (the amount after the reaction/the amount before the reaction) x 100, was measured. As a result, the residual rate after 24-hours reaction was 91.6%. From the result, is was revealed that cycloisomaltotetraose is a hardly digestible saccharide being only slightly degraded by small intestinal mucosal enzyme.

### Experiment 16

### Thermal stability of cycloisomaltotetraose

An aqueous cycloisomaltotetraose solution with a solid concentration of 7% (w/v) was prepared by dissolving the crystalline cycloisomaltotetraose preparation with a purity of 99.5% by weight, obtained in Experiment 3-1. Eight milliliters each of the aqueous solution was taken in a glass centrifuge tube with a screw cap, sealed, and then heated for 30 to 90 min at 120°C. The solutions before heating and those after heating for various times were subjected to the measurement for the degree of coloring and for the cycloisomaltotetraose purity by HPLC method. The degree of coloring was defined as the absorbance at 480 nm measured by using a 1-cm cell. The results are shown in Table 8.

**Table 8**

| Heating time (min) | Degree of coloring (A480nm) | Purity of cycloisomaltotetraose (% by weight) |
|---|---|---|
| 0 | 0.000 | 99.5 |
| 30 | 0.000 | 99.5 |
| 60 | 0.000 | 99.5 |
| 90 | 0.000 | 99.5 |

As is evident from the results in Table 8, aqueous solutions of cycloisomaltotetraose was not colored by heating at a high temperature, 120°C, and purity reduction due to decomposition was not also observed. Accordingly, it was found that cycloisomaltotetraose is a stable saccharide against heating.

### Experiment 17

### pH Stability of cycloisomaltotetraose

The crystalline cycloisomaltotetraose preparation obtained in Experiment 3-1 was dissolved in various buffers to make 7 kinds of aqueous cycloisomaltotetraose solutions with a cycloisomaltotetraose concentration of 4% (w/v), and with the pH adjusted to 3 to 9. Eight milliliters each of the aqueous solution was taken into a glass centrifuge tube with a screw cap, sealed, and heated at 100°C for 24 hours. After cooling them, the measurement for the degree of coloring degree of those aqueous solution and for the cycloisomaltotetraose purity were carried out in the same manner in Experiment 16. The results are shown in Table 9.

**Table 9**

| pH | Buffer | Degree of coloring (A480nm) | Purity of cycloisomaltotetraose (% by weight) |
|---|---|---|---|
| 3 | Acetate | 0.000 | 93.5 |
| 4 | Acetate | 0.000 | 99.4 |
| 5 | Acetate | 0.000 | 99.4 |
| 6 | Tris-HCl | 0.000 | 99.4 |
| 7 | Tris-HCl | 0.000 | 99.5 |
| 8 | Tris-HCl | 0.001 | 99.5 |
| 9 | Ammonium | 0.000 | 99.4 |

As is evident from Table 9, cycloisomaltotetraose was not substantially degraded in the range of pH 4 to 9 by heating at 100°C for 24 hours. Accordingly, it was found that cycloisomaltotetraose is stable in a wide range from weakly acidic to weakly alkaline conditions. However, it was slightly decomposed at pH 3, and a reduction of the purity was observed.

The following examples explain the present invention in detail. However, the present invention is not restricted by them.

### Example 1

A liquid medium (pH 6.8) containing 15 g/L of "DEXTRAN T70", a commercially available dextran commercialized by Pharmacosmos A/s., Denmark, 1.0 g/L of "POWDERY YEAST EXTRACT SH", a yeast extract commercialized by Nihon Pharmaceutical Co., Ltd., Tokyo, Japan, 5.0 g/L of "HIPOLYPEPTONE", a polypeptone commercialized by Nihon Pharmaceutical Co., Ltd., Tokyo, Japan, 1.0 g/L of dipotassium phosphate, 0.6 g/L of sodium phosphate dihydrate, 0.5 g/L of magnesium sulfate heptahydrate, 0.01 g/L of ferrous sulfate hexahydrate, 0.01 g/L of manganese sulfate pentahydrate, 3.0 g/L of calcium carbonate and water was placed in fifty 500 mL-Erlenmeyer flasks in respective amounts of 100 mL, sterilized by autoclaving at 121°C for 20 min, and cooled. Then, *Agreia* sp. D1110 (NITE BP-02815) was inoculated into the liquid medium and cultured by rotary-shaking the flasks at 240 rpm and at 27°C for 4 days. After completion of the cultivation, cells were removed by centrifuging the culture broth at 12,000 rpm for 10 min and culture supernatant was obtained. Then, about 4.8 L of the culture supernatant was concentrated using a UF-membrane and about 1,100 mL of a concentrated enzyme solution, containing 0.70 unit/mL of cycloisomaltotetraose-forming enzyme activity, was collected as a crude enzyme solution.

### Example 2

Except for replacing "POWDERY YEAST EXTRACT SH", a yeast extract commercialized by Nihon Pharmaceutical Co., Ltd., Tokyo, Japan, to "YEAST EXTRACT MEAST GRANULE S", a yeast extract commercialized by Asahi Food & Healthcare, Ltd., Tokyo, Japan; and "HIPOLYPEPTONE", a polypeptone commercialized by Nihon Pharmaceutical Co., Ltd., Tokyo, Japan, to "HINUTE S", commercialized by Fuji Oil Co., Ltd., Osaka, Japan, a liquid medium (pH 6.8) having the same composition with that in Example 1, was placed in fifty 500 mL-Erlenmeyer flasks in respective amounts of 100 mL, sterilized by autoclaving at 121°C for 20 min, and cooled. Then, *Microbacterium trichothecenolyticum* D2006 (NITE BP-02816) was inoculated into the liquid medium and cultured by rotary-shaking the flasks at 240 rpm and at 27°C for 4 days. After completion of the cultivation, cells were removed by centrifuging the culture broth at 12,000 rpm for 10 min and culture supernatant was obtained. Further, about 4.7 L of the culture supernatant was concentrated using a UF-membrane and about 920 mL of a concentrated enzyme solution, containing 0.41 unit/mL of cycloisomaltotetraose-forming enzyme activity, was collected as a crude enzyme solution.

### Example 3

Ten liters of 50 mM acetate buffer (pH 6.0) containing 2% (w/v) of "DEXTRAN T70", dextran commercialized by Pharmacosmos A/s., Denmark, and 0.006% (w/v) of hinokitiol as a preservative was admixed with 5 units/g-dextran of a crude enzyme solution containing cycloisomaltotetraose-forming enzyme from *Agreia* sp. D1110 (NITE BP-02815), obtained by the method of Example 1, and then incubated at 40°C for 24 hours. The reaction mixture containing 11.2% by weight, d.s.b., of cycloisomaltotetraose and 1.8% by weight, d.s.b., of isomaltotetraose was obtained. After inactivating the enzyme by heating the reaction mixture at 95°C for 30 min and cooling, high molecular substances were removed by filtrating the reaction mixture using a UF membrane with a molecular weight cut off of 10,000, and a saccharide solution (filtrate) containing 62% by weight, d.s.b., of cycloisomaltotetraose and 12.8% by weight, d.s.b., of isomaltotetraose was obtained. The obtained filtrate was purified by conventional methods, decoloring using activated charcoal and deionizing using ion-exchanger resins with H⁺ and OH⁻ forms, and further concentrated, and a syrup with a solid concentration of 60% by weight was obtained. The syrup as a saccharide solution was subjected to a column chromatography using "AMBERLITE CR-1310" (Na-form), a strongly acidic cation-exchange resin commercialized by Organo Corporation, Tokyo, Japan. The resin was packed into four jacketed stainless-steel columns having a diameter of 5.4 cm, which were then cascaded in series to give a total gel bed depth of 20 m. Under the conditions of keeping the inner column temperature at 60°C, the saccharide solution was fed to the columns in a volume of 5%(v/v) and fractionated by feeding to the columns hot water heated to 60°C at an SV (space velocity) of 0.13 to obtain fractions containing cycloisomaltotetraose. While monitoring the saccharide composition of elute by HPLC, and then the fractions containing cycloisomaltotetraose were collected and the fractions were concentrated. As a result, a cycloisomaltotetraose solution with a cycloisomaltotetraose purity of 98.6% by weight, d.s.b., was obtained. Successively, cycloisomaltotetraose was crystallized according to the method described in Experiment 3-1 and crystalline cycloisomaltotetraose pentahydrate with a purity of 99.8% by weight, d.s.b., was obtained. Since the product showed no reducing power, it can be advantageously used in various compositions such as foods and beverages, cosmetics, and pharmaceuticals as a quality-improving agent, stabilizer, excipient, base material for powderization, and the like.

### Example 4

Twenty liters of 50 mM acetate buffer (pH 6.0) containing 2.5% (w/v) of "DEXTRAN T70", dextran commercialized by Pharmacosmos A/s., Denmark, and 0.006% (w/v) of hinokitiol as a preservative was admixed with 5 units/g-dextran of a crude enzyme solution containing cycloisomaltotetraose-forming enzyme from *Microbacterium trichothecenolyticum* D2006 (NITE BP-02816), obtained by the method of Example 2, and then incubated at 35°C for 48 hours. The reaction mixture containing 9.7% by weight, d.s.b., of cycloisomaltotetraose and 2.0% by weight, d.s.b., of isomaltotetraose was obtained. After inactivating the enzyme by heating the reaction mixture at 95°C for 30 min and cooling, high molecular substances were removed by filtrating the reaction mixture using a UF membrane with a molecular weight cut off of 10,000, and a saccharide solution (filtrate) containing 62% by weight, d.s.b., of cycloisomaltotetraose and 12.8% by weight, d.s.b., of isomaltotetraose was obtained. The obtained saccharide solution was further concentrated and dried to make into a particulate composition containing cycloisomaltotetraose. The product contained 64% by weight, d.s.b., of cycloisomaltotetraose, 13% by weight, d.s.b., of isomaltotetraose, and 25% by weight, d.s.b., of other saccharides with a glucose polymerization degree of 7 or higher. The product can be advantageously used in various compositions such as foods and beverages, cosmetics, and pharmaceuticals as a quality-improving agent, stabilizer, excipient, base material for powderization, and the like.

### Example 5

A commercially available corn starch was suspended in 20 mM acetate buffer (pH 5.5) containing 1 mM calcium chloride to give a concentration of 30% by weight, dissolved by heating, and then cooled to 50°C. To the resulting material solution, α-glucosyl transferase disclosed in International Patent Application No. WO2008/136331 pamphlet, isoamylase (a starch debranching enzyme), and ""KLEISTASE E5CC", an α-amylase commercialized by Amano Enzyme, Aichi, Japan, were respectively admixed to give an enzyme amount of 10 units, 1,000 units, and 10 units per one gram solid of the substrate, and allowed to react at 50°C for 72 hours. Successively, the reaction mixture was heated at 100°C for 15 min to inactivate enzymes and a reaction mixture containing branched α-glucans having α-1,6 glucan as a partial structure was prepared.

A part of the reaction mixture containing branched α-glucans obtained above was diluted with 100 mM acetate buffer (pH 6.0) to give a concentration of 1.0 % by weight. To the diluted solution, 2.2 units/g-solid substrate of cycloisomaltotetraose-forming enzyme from *Microbacterium trichothecenolyticum* D2006 (NITE BP-02816) was admixed and allowed to react at 40°C for 48 hours. The reaction product contained 8.1% by weight of cycloisomaltotetraose, 5.7% by weight of isomaltotetraose, 4.2% by weight of isomaltotriose, and 5.0% by weight of isomaltose, d.s.b.. From the result, it was found that cycloisomaltotetraose can be produced from starchy substrate by using an enzyme which forms a saccharide having α-1,6 glucan as a partial structure from starch and cycloisomaltotetraose-forming enzyme in combination.

### Example 6

### Glucosyl derivatives of cycloisomaltotetraose

As an acceptor and a saccharide donor for a transglycosylation reaction, 0.5 g of crystalline cycloisomaltotetraose pentahydrate, obtained by the method in Example 3, and 2.5 g of "CAVAMAX W6 food", a commercially available α-cydodextrin commercialized by CycloChem Co., Ltd., Hyogo, Japan, were used, respectively. The acceptor and the saccharide donor were dissolved in 50 mL of 50 mM acetate buffer (pH 5.5) containing 1 mM calcium chloride to make into a substrate solution. The substrate solution was admixed with 2 units/g-saccharide donor of a glucanotransferase, CGTase from *Geobacillus stearothermophilus,* incubated at 50°C for 24 hours, and then heated at 100°C for 10 min to stop the enzyme reaction. Successively, the resulting reaction mixture was admixed with 10 units/g-saccharide donor of "GLUCOZYME #2000", a commercially available glucoamylase commercialized by Nagase ChemteX Corporation, Osaka, Japan, incubated at 50°C for 24 hours, and then heated at 100°C for 10 min to stop the enzyme reaction. To the resulting glucoamylase-treated solution, sodium hydroxide was admixed to adjust the pH to 13.0, and the resulting solution was subjected to alkaline treatment by autoclaving at 105 °C for one hour to degrade reducing sugars in the glucoamylase-treated solution. The resulting alkaline-treated solution was subjected to deionization, concentration and filtration, and a solution containing glucosyl-derivatives of cycloisomaltotetraose was obtained.

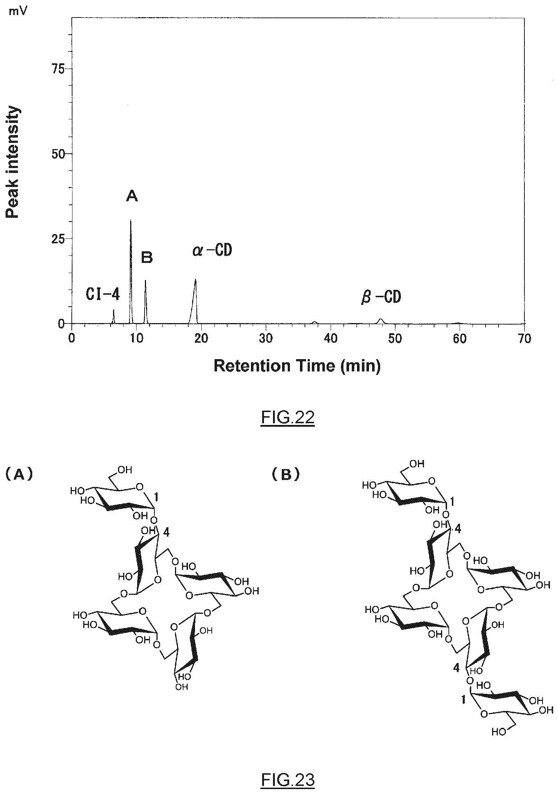

The saccharide solution containing glucosyl derivatives of cycloisomaltotetraose obtained above was subjected to HPLC analysis under the following conditions. The resulting chromatogram is shown in FIG. 22.

### (HPLC conditions)

Column: YMC-Pack ODS-AQ303 (4. 6 mm x 250 mm) (commercialized by YMC Co., Ltd., Kyoto, Japan);
Eluent: water/methanol (95:5, v/v);
Column temperature: 40°C;
Flow rate: 0. 7 mL/min;
Detection: Refractive index detector

As shown in FIG. 22, a peak of cycloisomaltotetraose was detected at a retention time of 6.47 min and two peaks considered to glucosyl-derivatives of cycloisomaltotetraose were detected at retention times of 9.12 min and 11.36 min. Further, a peak of α-cydodextrin used as a saccharide donor was detected at a retention time of 19.1 min and a peak of β-cyclodextrin considered to be formed from α-cydodextrin was detected at a retention time of 47.7 min. Saccharides eluted at retention times of 9.12 min and 11.36 min were designated to Glucosyl derivatives A and B, respectively. The contents of Glucosyl derivatives A and B in the saccharide solution containing glucosyl derivatives were 31.6% by weight, d.s.b. and 15.8% by weight, d.s.b., respectively.

The saccharide solution containing glucosyl derivatives was subjected to preparative HPLC using "D-ODS-5", an ODS column commercialized by YMC Co., Ltd., Kyoto, Japan, and Glucosyl derivatives A and B were collected. As the results, 229 mg of Glucosyl derivative A with a purity of 99.2% by weight, d.s.b., and 117 mg of Glucosyl derivative B with a purity of 98.0% by weight, d.s.b., were obtained. The preparations of Glucosyl derivatives A and B were subjected to LC/MS analysis and NMR analysis. From the analyses, it was revealed that the molecular mass of Glucosyl derivative A is 810 and Glucosyl derivative A is a penta-saccharide having a structure that one glucose molecule is bound to one of 4 glucose residues constructing cycloisomaltotetraose *via* α-1,4 glucosidic linkage, i.e., a mono-glucosyl derivative of cycloisomaltotetraose. It was also revealed that the molecular mass of Glucosyl derivative B is 972 and Glucosyl derivative B is a hexa-saccharide having a structure that two glucose molecules are bound to 2 glucose residues located diagonally in 4 glucose residues constructing cycloisomaltotetraose *via* α-1,4 glucosidic linkages, i.e., a di-glucosyl derivative of cycloisomaltotetraose. The structures of the glucosyl derivatives of cycloisomaltotetraose, Glucosyl derivatives A and B, are shown in FIG. 23.

### Example 7

### Hard candy

Thirty parts by weight of a crystalline cycloisomaltotetraose pentahydrate, obtained by the method in Example 3, was admixed with 100 parts by weight of sucrose solution with a sucrose concentration of 55% (w/v) with heating. Then, the mixture was concentrated under a reduced pressure to give a moisture content of less than 2%. The resulting concentrate was admixed with 0.6 part by weight of citric acid and an appropriate amount of lemon flavor and coloring, shaped into hard candy according to a conventional method. The product shows a satisfactory crisp, taste, flavor, and hardly causes the crystallization of sucrose due to containing cycloisomaltotetraose. The product is a high-quality hard candy with low hygroscopicity and no fluidity.

### Example 8

### Chocolate cookies

Using 140 parts by weight of flour (thin flour), 90 parts by weight of butter, 115 parts by weight of chocolate, 360 parts by weight of granulated sugar, 200 parts by weight of whole egg, 200 parts by weight of almond, and 30 parts by weight of powdery product containing cycloisomaltotetraose, prepared by the method of Example 4, chocolate cookies were produced by a conventional method. After 2 weeks storage at room temperature, the product was tasted. It was found that the product shows no moisture absorption and drying, and further, cycloisomaltotetraose suppresses oxidation and degradation of fats and oils and retained its flavor well right after production.

### Example 9

### Lactic acid bacteria beverage

One hundred seventy-five parts by weight of skim milk, 30 parts by weight of a powdery product containing cycloisomaltotetraose, obtained by the method in Example 4, and 70 parts by weight of "NYUKA-OLIGO", a lactosucrose high content powder commercialized by Hayashibara Co., Ltd, Okayama, Japan, were dissolved into 1,500 parts by weight of water, and then the resulting mixture was sterilized at 65°C for 30 min. After cooling the mixture to 40°C, 30 parts by weight of a lactic acid bacterium culture was inoculated to the mixture as a starter according to the conventional method, and cultured at 37°C for 8 hours to obtain a lactic acid bacteria beverage. The product has a satisfactory flavor and keeps the lactic acid bacterium stable because it comprises oligosaccharides and cycloisomaltotetraose. Further, the product is preferably used as a lactic acid bacteria beverage having a growth-promoting activity for bifidobacteria and a bowel-regulating property.

### Example 10

### Body soap

Fifteen parts by weight of potassium lauric acid, 5.0 parts by weight of potassium myristate, 4.0 parts by weight of a powdery product containing cycloisomaltotetraose, obtained by the method of Example 4, 2.0 parts by weight of propylene glycol, 0.5 part by weight of polyethylene powder, 0.5 part by weight of hydroxypropyl chitosan solution, 0.25 part by weight of glycine, 0.25 part by weight of glutamine, 0.1 part by weight of photosensitive dye No. 201, an appropriate amount of phenol, pH adjusting agent, and an appropriate amount of lavender water were mixed and then purified water was added to the mixture to give the total amount of 100 parts by weight, and successively a body soap was prepared by emulsifying the mixture by a conventional method. The product is a good foaming body soap with excellent feeling of use. Further, the product can be advantageously used for preventing the generation of body odor, preventing itching, or for the treating or preventing the aging of the skin. Further, the product has an excellent moisturizing property because it contains cycloisomaltotetraose, and it can be used without concern of hypersensitivity because it is less irritating to the skin.

### Example 11

### Cosmetic cream

According to conventional method, 2 parts by weight of polyoxyethylenglycol mono-stearate, 5 parts by weight of self-emulsified glycerin mono-stearate, 2 parts by weight of a crystalline cycloisomaltotetraose pentahydrate, obtained by the method in Example 3, one part by weight of "αG-RUTIN", α-glucosyl rutin, commercialized by Hayashibara Co. Ltd., Okayama, Japan, one part by weight of liquid paraffin, 10 parts by weight of glycerin-trioctanoate and an appropriate amount of preservative were mixed and dissolved by heating. The resulting mixture was further admixed with 2 parts by weight of L-lactic acid, 5 parts by weight of 1,3-butylen glycol, and 66 parts by weight of purified water, and the resulting mixture was emulsified using a homogenizer. The homogenized mixture was further admixed with an appropriate amount of flavor and stirred to make into a cosmetic cream. The product has an antioxidative activity of α-glucosyl rutin and said antioxidative activity is stabilized by cycloisomaltotetraose, therefore, it can be advantageously used as a sunburn preventive, skin-care agent and whitening agent for skin.

### Example 12

### Toothpaste

Forty-five parts by weight of calcium monohydrogen phosphate, 1.5 parts by weight of sodium lauryl sulfate, 25 parts by weight of glycerin, 0.5 part by weight of polyoxyethylene sorbitan laurate, 10 parts by weight of a crystalline cycloisomaltotetraose pentahydrate, obtained by the method in Example 3, 0.02 part by weight of saccharin, and 18 parts by weight of water were mixed to make into a toothpaste. The product is a toothpaste whose bad taste is improved and shows a satisfactory availability without losing the washing power of surfactant.

### INDUSTRIAL APPLICABILITY

According to the present invention, a novel saccharide, cycloisomaltotetraose, having a structure in which four D-glucose molecules are bound in cyclic form *via* α-1,6 glucosidic linkages, and a saccharide composition comprising the same can be produced and provided by using a cycloisomaltotetraose-forming enzyme. The present invention, which enables to provide a novel cycloisomaltotetraose, contributes to various fields such as foods and beverages, cosmetics, and pharmaceuticals, and its industrial significance is extremely large.

### EXPLANATION OF SYMBOLS

In FIG. 1,
   Lane 1: Isomaltooligosaccharides (marker);
   Lane 2: Isomaltotriose (standard product);
   Lane 3: Isomaltotetraose (standard product);
   Lane 4: Reaction products obtained by allowing a crude enzyme from a microorganism D1110 strain to act on a dextran substrate;
   G: Glucose;
   IG2: Isomaltose;
   IG3: Isomaltotriose;
   IG4: Isomaltotetraose;
   I: An unknown saccharide;
In FIGs. 4, 6 and 7,
   The dashed line is an auxiliary line added by the applicant for signal assignment.
   1H: Proton bound to the carbon at C1 position of D-glucose
   2H: Proton bound to the carbon at C2 position of D-glucose
   3H: Proton bound to the carbon at C3 position of D-glucose
   4H: Proton bound to the carbon at C4 position of D-glucose
   5H: Proton bound to the carbon at C5 position of D-glucose
   6Ha and 6Hb: Protons bound to the carbon at C6 position of D-glucose
In FIGs 6 and 7,
   1C: Carbon at C1 position of D-glucose
   2C: Carbon at C2 position of D-glucose
   3C: Carbon at C3 position of D-glucose
   4C: Carbon at C4 position of D-glucose
   5C: Carbon at C5 position of D-glucose
   6C: Carbon at C6 position of D-glucose
In FIG. 10,
   ↓: Characteristic diffraction peaks in the powder X-ray diffraction pattern of crystalline cycloisomaltotetraose pentahydrate.
In FIG. 19,
   ORF9038: Amino acid sequence encoded by cycloisomaltotetraose-forming enzyme gene derived from *Agreia* sp. D1110.
   ORF5328: Amino acid sequence encoded by cycloisomaltotetraose-forming enzyme gene derived from *Microbacterium trichothecenolyticum* D2006.
In FIG. 20,
   f1 ori: f1 phage replication origin;
   Ampicillin: Ampicillin resistance gene;
   pUC ori: pUC replication origin;
   P_{T7}: T7 promoter;
   RBS: Ribosome binding site;
   ATG: Initial codon;
   (Black arrow): cycloisomaltotetraose-forming enzyme gene;
In FIG. 21,
   Dex: Dextran;
   Cl-4: Cycloisomaltotetraose;
In FIG. 22,
   Cl-4: Cycloisomaltotetraose;
   A: Cycloisomaltotetraose derivative A;
   B: Cycloisomaltotetraose derivative B;
   α-CD: α-Cyclodextrin:
   β-CD: β-Cyclodextrin;
In FIG. 23
   (A) Derivative A: A glucosyl derivative of cycloisomaltotetraose;
   (B) Derivative B: A di-glucosyl derivative of cycloisomaltotetraose;

## Claims

1. A cycloisomaltotetraose, having a structure in which 4 D-glucose molecules are bound in cyclic form *via* α-1,6 glucosidic linkages.

2. A crystal of the cycloisomaltotetraose of claim 1.

3. The crystal of claim 2, which is in the form of a pentahydrate crystal.

4. The crystal of claim 3, exhibiting characteristic diffraction peaks at the diffraction angles (2θ) of 9.0°, 9.8°, 11.8°, 13.1° and 19.1° in its powder X-ray diffraction pattern.

5. A saccharide composition comprising the cycloisomaltotetraose, which comprises the cycloisomaltotetraose of claim 1 and/or the crystal of claim 2, together with other saccharide(s).

6. The saccharide composition comprising the cycloisomaltotetraose of claim 5, which is in the form of syrup, powder, crystal-containing powder, or solid.

7. A glucosyl derivative of the cycloisomaltotetraose, having a structure in which one or two D-glucose molecules are bound to the cycloisomaltotetraose.

8. A cycloisomaltotetraose-forming enzyme which has an activity of acting on α-1,6 glucan with a glucose polymerization degree of 4 or higher, dextran, or partial hydrolysate of dextran and forming the cycloisomaltotetraose of claim 1.

9. The cycloisomaltotetraose-forming enzyme of claim 8, which has the following physicochemical properties:
(1) Molecular weight
86,000 ± 10,000 daltons on SDS-polyacrylamide gel electrophoresis;
(2) Optimum temperature
35 to 40°C when reacted at pH 6.0 for 30 min;
(3) Optimum pH
pH 5.5 to 6.0 when reacted at 40°C for 30 min;
(4) Thermal stability
Stable up to the temperature of 40°C when incubated at pH 6.0 for 30 min; and
(5) pH Stability
Stable in a range of pH 5.0 to 10.0 when incubated at 4°C for 16 hours.

10. The cycloisomaltotetraose-forming enzyme of claim 8 or 9, having an amino acid sequence of SEQ ID NO: 5 or SEQ ID NO: 6 as the N-terminal amino acid sequence.

11. The cycloisomaltotetraose-forming enzyme of any one of claims 8 to 10, which has an amino acid sequence of SEQ ID NO: 7 or SEQ ID NO: 8, or an amino acid sequence where one or more amino acid residues in SEQ ID NO: 7 or SEQ ID NO: 8 are deleted, replaced, or added without altering the enzyme activity.

12. The cycloisomaltotetraose-forming enzyme of claim 11, which has an amino acid sequence with the homology of 70% or higher against the amino acid sequence of SEQ ID NO: 7 or SEQ ID NO: 8.

13. The cycloisomaltotetraose-forming enzyme of any one of claims 8 to 12, which is derived from a microorganism.

14. The cycloisomaltotetraose-forming enzyme of claim 13, wherein said microorganism belongs to the genus *Agreia* or the genus *Microbacterium.*

15. The cycloisomaltotetraose-forming enzyme of claim 14, wherein said microorganism belonging to the genus *Agreia* is *Agreia* sp. D1110 (NITE Patent Microorganisms Depositary, National Institute of Technology and Evaluation, Accession No. NITE BP-02815) or a mutant thereof, and said microorganism belonging to the genus *Microbacterium* is *Microbacterium trichothecenolyticum* D2006 (NITE Patent Microorganisms Depositary, National Institute of Technology and Evaluation, Accession No. NITE BP-02816) or a mutant thereof.

16. A microorganism capable of producing the cycloisomaltotetraose-forming enzyme of any one of claims 8 to 15, which is *Agreia* sp. D1110 (NITE Patent Microorganisms Depositary, National Institute of Technology and Evaluation, Accession No. NITE BP-02815) or a mutant thereof or *Microbacterium trichothecenolyticum* D2006 (NITE Patent Microorganisms Depositary, National Institute of Technology and Evaluation, Accession No. NITE BP-02816) or a mutant thereof.

17. A DNA, which encodes the cycloisomaltotetraose-forming enzyme of claim 11.

18. The DNA of claim 17, which comprises a nucleotide sequence of SEQ ID NO: 9 or SEQ ID NO: 10, a nucleotide sequence where one or more nucleotides in SEQ ID NO: 9 or SEQ ID NO: 10 are deleted, replaced, or added without altering the encoded enzyme activity, or complementary nucleotide sequences thereof.

19. The DNA of claim 18, which is obtainable by replacing one or more nucleotides of SEQ ID NO: 9 or SEQ ID NO: 10 without altering the amino acid sequence encoded thereby based on the degeneracy of genetic code.

20. The DNA of any one of claims 17 to 19, which is derived from a microorganism of the genus *Agreia* or the genus *Microbacterium.*

21. A replicable recombinant DNA, which comprises the DNA of any one of claims 17 to 20 and an autonomously replicable vector.

22. A transformant, which is obtainable by introducing the recombinant DNA of claim 21 into an appropriate host.

23. A process for producing a cycloisomaltotetraose-forming enzyme, comprising the steps of:
culturing a microorganism capable of producing the cycloisomaltotetraose-forming enzyme of any one of claims 8 to 15 in a nutrient culture medium; and
collecting the cycloisomaltotetraose-forming enzyme from the resulting culture.

24. A process for producing a recombinant cycloisomaltotetraose-forming enzyme, comprising the steps of:
culturing the transformant of claim 22; and
collecting a recombinant cycloisomaltotetraose-forming enzyme from the resulting culture.

25. A process for producing the cycloisomaltotetraose of claim 1, the crystal of the cycloisomaltotetraose of claim 2, or the saccharide composition comprising cycloisomaltotetraose of claim 5 or 6, comprising a step of allowing the cycloisomaltotetraose-forming enzyme of any one of claims 8 to 15 to act on a solution containing α-1,6 glucan with a glucose polymerization degree of 4 or higher, dextran, or a partial hydrolysate of dextran.

26. A process for producing the cycloisomaltotetraose of claim 1, the crystal of the cycloisomaltotetraose of claim 2, or the saccharide composition comprising cycloisomaltotetraose of claim 5 or 6, comprising a step of allowing an enzyme, which has an activity of acting on starch or a partial starch hydrolysate and forming α-1,6 glucan with a glucose polymerization degree of 4 or higher, dextran, or a saccharide having α-1,6 glucan structure as a part; and the cycloisomaltotetraose-forming enzyme of any one of claims 8 to 15 to act in combination on a solution containing starch or a partial starch hydrolysate.

27. A composition comprising the cycloisomaltotetraose of claim 1, the crystal of the cycloisomaltotetraose of claim 2, the saccharide composition comprising cycloisomaltotetraose of claim 5 or 6, or the glucosyl derivative of cycloisomaltotetraose of claim 7, together with other ingredient(s).

28. The composition of claim 27, which is in the form of foods and beverages, luxury grocery items, feeds, baits, cosmetics, pharmaceuticals, or industrial supplies.


SEQUENCE LISTING

<110>  Hayashibara Co., Ltd.

<120>  Cycloisomaltotetraose, cycloisomaltotetraose-forming enzyme, their
preparation and uses

<130>  10130502

<160>  12

<210>  1
<211>  20
<212>  DNA
<213>  Artificial Sequence

<400>  1
AGAGTTTGAT CATGGCTCAG                                                20

<210>  2
<211>  22
<212>  DNA
<213>  Artificial Sequence

<400>  2
TACGGTTACC TTGTTACGAC TT                                             22


<210>  3
<211>  1447
<212>  DNA
<213>  Unknown

<400>  3
GACGAACGCT GGCGGCGTGC TTAACACATG CAAGTCGAAC GATGAAGCTC CAGCTTGCTG    60

GGGTGGATTA GTGGCGAACG GGTGAGTAAC ACGTGAGTAA CCTGCCCTTG ACTCTGGGAT   120

AAGCGTTGGA AACGACGTCT AATACCGGAT ATGACGACCG ATGGCATCAT CTGGTTGTGG   180

AAAGAATTTT GGTCAAGGAT GGACTCGCGG CCTATCAGGT AGTTGGTGAG GTAATGGCTC   240

ACCAAGCCTA CGACGGGTAG CCGGCCTGAG AGGGTGACCG GCCACACTGG GACTGAGACA   300

CGGCCCAGAC TCCTACGGGA GGCAGCAGTG GGGAATATTG CACAATGGGC GAAAGCCTGA   360

TGCAGCAACG CCGCGTGAGG GATGACGGCC TTCGGGTTGT AAACCTCTTT TAGTAGGGAA   420

GAAGCGAAAG TGACGGTACC TGCAGAAAAA GCACCGGCTA ACTACGTGCC AGCAGCCGCG   480

GTAATACGTA GGGTGCAAGC GTTGTCCGGA ATTATTGGGC GTAAAGAGCT CGTAGGCGGT   540

TTGTCGCGTC TGCTGTGAAA ACTGGAGGCT CAACCTCCAG CCTGCAGTGG GTACGGGCAG   600

ACTAGAGTGC GGTAGGGGAG ATTGGAATTC CTGGTGTAGC GGTGGAATGC GCAGATATCA   660

GGAGGAACAC CGATGGCGAA GGCAGATCTC TGGGCCGTAA CTGACGCTGA GGAGCGAAAG   720

CATGGGGAGC GAACAGGATT AGATACCCTG GTAGTCCATG CCGTAAACGT TGGGAACTAG   780

ATGTAGGGGC CATTCCACGG TTTCTGTGTC GCAGCTAACG CATTAAGTTC CCCGCCTGGG   840

GAGTACGGCC GCAAGGCTAA AACTCAAAGG AATTGACGGG GGCCCGCACA AGCGGCGGAG   900

CATGCGGATT AATTCGATGC AACGCGAAGA ACCTTACCAA GGCTTGACAT ATACGAGAAC 960

GGGCTAGAAA TAGTCAACTC TTTGGACACT CGTAAACAGG TGGTGCATGG TTGTCGTCAG 1020

CTCGTGTCGT GAGATGTTGG GTTAAGTCCC GCAACGAGCG CAACCCTCGT TCTATGTTGC 1080

CAGCACGTTA TGGTGGGAAC TCATAGGAGA CTGCCGGGGT CAACTCGGAG GAAGGTGGGG 1140

ATGACGTCAA ATCATCATGC CCCTTATGTC TTGGGCTTCA CGCATGCTAC AATGGCCGGT 1200

ACAAAGGGCT GCGATACCGT AAGGTGGAGC GAATCCCAAA AAGCCGGTCT CAGTTCGGAT 1260

TGAGGTCTGC AACTCGACCT CATGAAGTCG GAGTCGCTAG TAATCGCAGA TCAGCAACGC 1320

TGCGGTGAAT ACGTTCCCGG GCCTTGTACA CACCGCCCGT CAAGTCATGA AAGTCGGTAA 1380

CACCCAACGC CAGTGGCCTA ACCCGTAAGG GAGGGAGCTG TCTAAGGTGG GATCGGTGAT 1440

TAGGACT 1447

<210> 4
<211> 1485
<212> DNA
<213> Unknown

<400> 4
GAGTTTGATC ATGGCTCAGG ATGAACGCTG GCGGCGTGCT TAACACATGC AAGTCGAACG 60

GTGAAGCCAA GCTTGCTTGG TGGATCAGTG GCGAACGGGT GAGTAACACG TGAGCAACCT 120

GCCCTGGACT CTGGGATAAG CGCTGGAAAC GGCGTCTAAT ACTGGATATG AGCTCTCATC 180

GCATGGTGGG GGTTGGAAAG ATTTTTTGGT CTGGGATGGG CTCGCGGCCT ATCAGCTTGT 240

TGGTGAGGTA ATGGCTCACC AAGGCGTCGA CGGGTAGCCG GCCTGAGAGG GTGACCGGCC 300

ACACTGGGAC TGAGACACGG CCCAGACTCC TACGGGAGGC AGCAGTGGGG AATATTGCAC 360

AATGGGCGGA AGCCTGATGC AGCAACGCCG CGTGAGGGAT GACGGCCTTC GGGTTGTAAA 420

CCTCTTTTAG CAAGGAAGAA GCTTTTGTGA CGGTACTTGC AGAAAAAGCG CCGGCTAACT 480

ACGTGCCAGC AGCCGCGGTA ATACGTAGGG CGCAAGCGTT ATCCGGAATT ATTGGGCGTA 540

AAGAGCTCGT AGGCGGTTTG TCGCGTCTGC TGTGAAATCC CGAGGCTCAA CCTCGGGCCT 600

GCAGTGGGTA CGGGCAGACT AGAGTGCGGT AGGGGAGATT GGAATTCCTG GTGTAGCGGT 660

GGAATGCGCA GATATCAGGA GGAACACCGA TGGCGAAGGC AGATCTCTGG GCCGTAACTG 720

ACGCTGAGGA GCGAAAGGGT GGGGAGCAAA CAGGCTTAGA TACCCTGGTA GTCCACCCCG 780

TAAACGTTGG GAACTAGTTG TGGGGTCCTT TCCACGGATT CCGTGACGCA GCTAACGCAT 840

TAAGTTCCCC GCCTGGGGAG TACGGCCGCA AGGCTAAAAC TCAAAGGAAT TGACGGGGAC 900

CCGCACAAGC GGCGGAGCAT GCGGATTAAT TCGATGCAAC GCGAAGAACC TTACCAAGGC 960

TTGACATACA CGAGAACATC CCAGAAATGG GGGACTCTTT GGACACTCGT GAACAGGTGG 1020

TGCATGGTTG TCGTCAGCTC GTGTCGTGAG ATGTTGGGTT AAGTCCCGCA ACGAGCGCAA 1080

CCCTCGTTCT ATGTTGCCAG CACGTAATGG TGGGAACTCA TGGGATACTG CCGGGGTCAA 1140

CTCGGAGGAA GGTGGGGATG ACGTCAAATC ATCATGCCCC TTATGTCTTG GGCTTCACGC 1200

ATGCTACAAT GGCCGGTACA AAGGGCTGCA ATACCGTGAG GTGGAGCGAA TCCCAAAAAG 1260

CCGGTCCCAG TTCGGATTGA GGTCTGCAAC TCGACCTCAT GAAGTCGGAG TCGCTAGTAA 1320

TCGCAGATCA GCAACGCTGC GGTGAATACG TTCCCGGGTC TTGTACACAC CGCCCGTCAA 1380

GTCATGAAAG TCGGTAACAC CTGAAGCCGG TGGCCCAACC CTTGTGGAGG GAGCCGTCGA 1440

AGGTGGGATC GGTAATTAGG ACTAAGTCGT AACAAGGTAA CCGTA 1485

<210> 5
<211> 15
<212> PRT
<213> Agreia sp.

<400> 5
Ala Thr Val Gly Asp Ala Trp Thr Asp Lys Ala Arg Tyr Asp Pro
1               5                   10                  15

<210> 6
<211> 9
<212> PRT
<213> Microbacterium trichothecenolyticum

<400> 6
Ala Asp Leu Gly Asp Ala Trp Thr Asp
1               5

<210> 7
<211> 810
<212> PRT
<213> Agreia sp.

<400> 6
Ala Thr Val Gly Asp Ala Trp Thr Asp Lys Ala Arg Tyr Asp Pro Gly
1               5                   10                  15
Gln Gln Val Thr Val Thr Ala Glu Val Asp Gly Ser Gly Pro Val Glu
            20                  25                  30
Phe Ser Leu Val His Leu Gly Glu Thr Val Arg Thr Gly Thr Val Gln
        35                  40                  45
Ala Thr Gly Ser Gly Thr Val Thr Trp Thr Val Thr Pro Pro Thr Thr
    50                  55                  60
Asp Phe Thr Gly Tyr Leu Val His Ala Asp Ala Gly Gly Ser Thr Ala
65                  70                  75                  80
Gln Thr Ala Val Asp Val Ser Ser Thr Trp Thr Arg Phe Pro Arg Met
                85                  90                  95
Gly Tyr Leu Asp Glu Tyr Pro Ala Asp Ser Thr Gln Ala Asp Arg Asp
            100                 105                 110
Ala Ile Val Thr Asp Leu Ala Asn Lys Tyr His Leu Asn Ala Leu Gln
        115                 120                 125
Phe Tyr Asp Trp Met Trp Arg His Glu Ala Pro Ile Glu Arg Asp Gly
    130                 135                 140
Asn Gly Asp Leu Val Gly Thr Trp Thr Ala Trp Asn Gly Asp Val Ile
145                 150                 155                 160
Ala Pro Ala Thr Val Ser Gly Leu Val Asp Ala Gly His Ala Val Asn
                165                 170                 175
Val Ala Ala Leu Pro Tyr Ser Met Ser Tyr Ala Ala Leu Glu Gly Phe

            180                 185                 190
Glu Ala His Gly Val Asp Ala Asp Trp Arg Leu Lys Tyr Arg Ser Asp
        195                 200                 205
Ser Ser Asp Trp Lys Phe Gln Met Leu Pro Gly Arg Pro Asp Thr Asn
    210                 215                 220
Leu Trp Ile Met Asn Pro Ser Asn Pro Asp Trp Arg Asp His Ile Ser
225                 230                 235                 240
Ala Glu Tyr Gln Asp Gln Ile Asp Thr Phe Gly Phe Asp Gly Thr His
                245                 250                 255
Leu Asp Gln Leu Gly Asn Trp Gly Ser Gly Ala Ser Asp Gly Gly Met
            260                 265                 270
Asn Asp Val Ala Gly Asn Pro Val Asp Ile Pro Val Gly Phe Arg Asp
        275                 280                 285
Leu Val Ala Glu Thr Lys Ala Ala Thr Gly Lys Pro Val Gly Phe Asn
    290                 295                 300
Ala Val Asp Gly Phe Ala Gly Ser Thr Leu Ala Ser Ser Ser Ser Asp
305                 310                 315                 320
Tyr Leu Tyr Thr Glu Leu Trp Glu Asn His Glu Thr Tyr Ser Glu Val
                325                 330                 335
Gln Ser Tyr Leu Ala Gly Gln Arg Ala Glu Ser Gly Gly Lys Ala Ala
            340                 345                 350
Val Thr Ala Ala Tyr Leu Asn Tyr Arg Ser Asn Thr Gly Asp Arg Tyr
        355                 360                 365
Glu Ala Glu Asp Gly Thr Leu Ile Gly Val Gln Val Asn Ser Asn His
    370                 375                 380
Pro Gly Tyr Thr Gly Thr Gly Phe Val Asp Gln Phe Gly Asp Val Gly
385                 390                 395                 400
Asp Ser Val Thr Leu Ser Val Thr Val Pro Glu Ser Arg Arg Tyr Gly
                405                 410                 415
Ile Val Pro Ala Trp Ala Asn Asp Thr Ala Thr Thr Ala Thr Arg Thr
            420                 425                 430
Val Arg Val Asp Gly Val Pro Ala Gly Lys Leu Lys Met Thr Pro Thr
        435                 440                 445
Gly Ser Trp Asp Ser Trp Asn Thr Glu Ala Gly Phe Gly Thr Tyr Leu
    450                 455                 460
Ser Ala Gly Ser His Thr Ile Glu Val Ser Leu Asp Ala Gly Asp Thr
465                 470                 475                 480
Gly Phe Ala Asn Leu Asp Ser Ile Thr Leu Gly Thr Phe Asn Thr Pro
                485                 490                 495
Ser Val Gln Leu Ala Asn Ala Ala Leu Ala Ala Ser Gly Ala Ser His
            500                 505                 510
Ile Glu Met Gly Gln Gly Asp Gln Met Leu Val Ala Pro Tyr Phe Leu
        515                 520                 525
Asp Asp Ser Lys Gln Met Ser Thr Glu Leu Gln Ala Trp Met Glu Ser
    530                 535                 540
Tyr Tyr Asp Val Ile Thr Gly Tyr Glu Asn Leu Leu Tyr Gly Pro Thr
545                 550                 555                 560
Leu Arg Gln Leu Pro Asn Ala Val Ser Ile Ala Gly His Thr Thr Ser
                565                 570                 575
Thr Asp Gly Thr Gly Asp Thr Ile Trp Ala Thr Pro Met Arg Asn Asp
            580                 585                 590
Gly Met Asp Val Leu His Leu Ile Asn Leu Glu Gly Asn Asp Gly Arg
        595                 600                 605
Trp Arg Glu Pro Ala Ala Gln Ala Pro Thr Leu Ser Asn Leu Pro Val
    610                 615                 620
Lys Tyr Tyr Leu Ala Asp Arg Pro Val Pro Ala Asp Val Arg Ile Ala
625                 630                 635                 640
Ser Pro Asp Asn Gly Gly Arg Ser Ser Thr Leu Ala Phe Thr Ser Gly
                645                 650                 655
Ser Asp Ala Gly Gly Asn Tyr Ile Ser Phe Thr Val Pro Glu Leu Lys
            660                 665                 670
Asn Trp Ser Phe Val Tyr Leu Gly Asp Ser Thr Arg Gly Asn Gly Asn
        675                 680                 685

Val Val Thr Gln Ala Ser Thr Cys Leu Asp Val Ala Gly Gly Asn Ser
    690                 695                 700
Ala Asp Gly Thr Ala Val Gln Val Trp Asn Cys Ala Ser Val Pro Ala
705                 710                 715                 720
Met Gln Trp Thr Phe Gln Asn Asn Thr Leu Ser Ala Leu Gly Lys Cys
                725                 730                 735
Leu Asp Leu Val Ala Gly Gly Thr Ala Asp Gly Thr Leu Ala His Leu
            740                 745                 750
Trp Thr Cys Ser Asp Val Pro Ser Gln Thr Trp Glu Arg Thr Gly Gln
        755                 760                 765
Gly Gln Tyr Leu Asn Pro Ala Ser Gly Lys Cys Leu Asp Leu Val Gly
    770                 775                 780
Gly Ser Thr Ala Asn Gly Thr Arg Ala His Leu Trp Ser Cys His Ala
785                 790                 795                 800
Gly Ala Ser Gln Lys Trp Thr Leu Pro Gln
                805                 810

<210> 8
<211> 811
<212> PRT
<213> Microbacterium trichothecenolyticum

<400> 8
Ala Asp Leu Gly Asp Ala Trp Thr Asp Lys Ala Arg Tyr Ala Pro Gly
1               5                   10                  15
Glu Gln Val Thr Val Thr Ala Glu Val Ser Gly Thr Gly Ser Val Gln
            20                  25                  30
Phe Ser Leu Val His Leu Gly Asp Val Val Asp Thr Gly Thr Val Thr
        35                  40                  45
Ala Thr Gly Ser Gly Gln Val Asn Trp Thr Val Thr Pro Pro Ala Asn
    50                  55                  60
Asp Phe Thr Gly Tyr Met Val His Val Asp Ala Gly Ser Ser Thr Thr
65                  70                  75                  80
Gln Thr Ala Val Asp Val Ser Ser Asn Trp Thr Arg Phe Pro Arg Thr
                85                  90                  95
Gly Phe Leu Asp Thr Tyr Pro Ile Asp Leu Asp Ser Thr Glu Gln Ala
            100                 105                 110
Ala Thr Val Asp Glu Leu Ser Arg Asn Tyr His Leu Asn Ser Leu Gln
        115                 120                 125
Phe Tyr Asp Trp Met Trp Arg His Glu Lys Pro Val Gln Arg Glu Ser
    130                 135                 140
Asn Gly Asp Leu Val Asp Thr Trp Thr Ala Trp Asn Gly Asp Val Ile
145                 150                 155                 160
Ala Pro Asp Thr Val Ala Gly Leu Ile Asp Ala Thr His Asp Val Asn
                165                 170                 175
Ala Ala Ala Leu Pro Tyr Ser Met Ser Tyr Ala Ala Leu Glu Gly Phe
            180                 185                 190
Gln Ala His Gly Val Asp Ala Asp Trp Arg Leu Lys Tyr Arg Ser Ser
        195                 200                 205
Gly Asp Asp Trp Lys Phe Gln Met Leu Pro Asn Glu Pro Asp Thr Thr
    210                 215                 220
Leu Trp Ile Met Asn Pro Glu Asn Pro Asp Trp Val Ser His Ile Thr
225                 230                 235                 240
Glu Gln Tyr Ala Asp Gln Ile Asp Ala Leu Gly Phe Asp Gly Thr His
                245                 250                 255
Ile Asp Gln Leu Gly Asn Trp Gly Glu Ala Val Asp Gly Gly Met Asp
            260                 265                 270
Asp Val Ala Gly Asp Pro Val Asp Ile Pro Gln Gly Phe Ala Asn Leu
        275                 280                 285
Val Ala Ala Thr Lys Asp Ala Thr Gly Lys Thr Thr Gly Phe Asn Ala
    290                 295                 300
Val Asp Gly Phe Gly Ser Asp Trp Leu Ala Gly Ser Glu Ser Asp Tyr
305                 310                 315                 320

Leu Tyr Thr Glu Leu Trp Glu Asn His Glu Thr Tyr Ala Gln Val Gln
                325                 330                 335
Asp Tyr Leu Ala Gln Gln Arg Ala Ala Ser Gly Gly Lys Gly Ala Val
            340                 345                 350
Leu Ala Ala Tyr Leu Asn Tyr Arg Ser Asn Thr Gly Asp Gln Tyr Glu
        355                 360                 365
Ala Glu Ala Gly Val Leu Ala Gly Gly Val Gln Val Asp Ser Asp His
    370                 375                 380
Thr Gly Tyr Thr Gly Thr Gly Phe Ile Asp Ser Tyr Gly Ala Ser Gly
385                 390                 395                 400
Asp Ala Val Thr Val Thr Val Ala Val Pro Glu Ser Arg Arg Tyr Gly
                405                 410                 415
Ile Val Pro Arg Trp Thr Asn Gly Thr Gly Ala Thr Ala Thr Arg Thr
            420                 425                 430
Val Thr Val Asp Gly Thr Asn Val Gly Arg Ile Lys Leu Pro Ser Thr
        435                 440                 445
Ala Asp Trp Asp Thr Trp Asn Ile Glu Gly Gly Thr Ala Ala Tyr Leu
    450                 455                 460
Thr Ala Gly Thr His Thr Ile Thr Leu Ala Val Gly Ser Gly Asp Thr
465                 470                 475                 480
Gly Tyr Val Asn Leu Asp Ser Val Thr Leu Gly Thr Phe Asp Thr Pro
                485                 490                 495
Ser Val Gln Leu Ala Asn Ala Ala Phe Ala Ala Asn Gly Ala Ser His
            500                 505                 510
Ile Glu Met Gly Gln Gly Asp Gln Met Leu Val Ala Pro Tyr Phe Leu
        515                 520                 525
Asp Glu Thr Lys Gln Met Asn His Glu Leu Gln Gly Trp Leu Glu Lys
    530                 535                 540
Tyr Tyr Asp Val Ile Thr Gly Tyr Glu Asn Leu Phe Tyr Gly Pro Thr
545                 550                 555                 560
Leu Arg Gln Leu Ser Asn Ala Val Thr Ile Ser Gly Gln Pro Thr Ser
                565                 570                 575
Thr Asp Gly Ala Ala Asn Thr Val Trp Thr Asn Val Met Arg Asn Asp
            580                 585                 590
Gly Ile Asp Val Ile His Leu Ile Asn Leu Lys Gly Asn Asn Gly Ser
        595                 600                 605
Trp Arg Asp Pro Ala Ala Thr Pro Ser Thr Leu Thr Asn Leu Pro Val
    610                 615                 620
Lys Tyr Tyr Leu Gly Asp Ser Pro Val Pro Ala Ser Val His Val Ala
625                 630                 635                 640
Ser Pro Asp Arg Asp Gly Gly Arg Ser Thr Ala Leu Pro Phe Thr Thr
                645                 650                 655
Gly Ser Asp Ala Asn Gly Thr Tyr Leu Ser Phe Thr Val Pro Glu Leu
            660                 665                 670
Lys Ser Trp Asp Phe Val Tyr Leu Gly Asp Ser Thr Ala Gly Asn Gly
        675                 680                 685
Asn Val Val Thr Lys Ala Ser Lys Cys Leu Asp Val Ala Gly Gly Ser
    690                 695                 700
Ala Ala Asn Gly Thr Ala Val Gln Ile Trp Asp Cys Ala Ser Val Pro
705                 710                 715                 720
Ala Gln Asp Trp Asp Tyr Ala Gly Glu Lys Leu Ser Ala Leu Gly Lys
                725                 730                 735
Cys Leu Asp Leu Tyr Gln Gly Gly Thr Ala Asn Gly Thr Leu Ala His
            740                 745                 750
Leu Trp Asp Cys Ala Asn Val Pro Ser Gln Gln Trp Val Arg Thr Ala
        755                 760                 765
Gln Ser Gln Tyr Tyr Asn Pro Ala Ser Gly Arg Cys Leu Asp Thr Val
    770                 775                 780
Gly Gly Gly Ala Ala Asn Gly Thr Arg Ile His Leu Trp Asp Cys His
785                 790                 795                 800
Ala Gly Pro Ser Gln Lys Trp Thr Leu Pro Gly
                805                 810

<210>  9
<211>  2523
<212>  DNA
<213>  Agreia sp.

<400>  9
atg aaa tct cgt ttt cgt cgc acg ctg ggg gcg tgc ggc acg gca gca      48
Met Lys Ser Arg Phe Arg Arg Thr Leu Gly Ala Cys Gly Thr Ala Ala
1               5                   10                  15
ttg ata gcg gcg ggg gtt gtg ctt ccc gcg gtg ccc gcg tcc gcg gca      96
Leu Ile Ala Ala Gly Val Val Leu Pro Ala Val Pro Ala Ser Ala Ala
            20                  25                  30
acc gtc ggc gac gcg tgg acc gac aag gcc cgt tac gat ccg ggg cag      144
Thr Val Gly Asp Ala Trp Thr Asp Lys Ala Arg Tyr Asp Pro Gly Gln
        35                  40                  45
cag gtc acg gtc acg gcc gag gtc gac ggg agt ggc ccc gtc gag ttc      192
Gln Val Thr Val Thr Ala Glu Val Asp Gly Ser Gly Pro Val Glu Phe
    50                  55                  60
tcg ctc gtg cat ctc ggc gag acc gtg agg acc ggg acc gtg cag gcg      240
Ser Leu Val His Leu Gly Glu Thr Val Arg Thr Gly Thr Val Gln Ala
65                  70                  75                  80
acc ggt tcg gga acc gtg acg tgg acg gtg aca cct ccc aca acg gac      288
Thr Gly Ser Gly Thr Val Thr Trp Thr Val Thr Pro Pro Thr Thr Asp
                85                  90                  95
ttt acg ggt tac ctg gtg cac gcc gac gcg gga ggc tca acg gcc cag      336
Phe Thr Gly Tyr Leu Val His Ala Asp Ala Gly Gly Ser Thr Ala Gln
            100                 105                 110
acc gcc gtc gat gtg tcg agc acc tgg acc cga ttc ccg cga atg ggc      384
Thr Ala Val Asp Val Ser Ser Thr Trp Thr Arg Phe Pro Arg Met Gly
        115                 120                 125
tac ctg gac gag tat ccg gcc gac agc acg cag gcg gat cgc gac gca      432
Tyr Leu Asp Glu Tyr Pro Ala Asp Ser Thr Gln Ala Asp Arg Asp Ala
    130                 135                 140
atc gtg acg gac ctc gcc aac aag tac cac ttg aac gcg ctg cag ttc      480
Ile Val Thr Asp Leu Ala Asn Lys Tyr His Leu Asn Ala Leu Gln Phe
145                 150                 155                 160
tac gac tgg atg tgg cgg cac gaa gct ccc atc gag cgc gac ggc aac      528
Tyr Asp Trp Met Trp Arg His Glu Ala Pro Ile Glu Arg Asp Gly Asn
                165                 170                 175
ggc gat ctg gtc ggc acc tgg acg gcg tgg aac ggc gat gtg atc gca      576
Gly Asp Leu Val Gly Thr Trp Thr Ala Trp Asn Gly Asp Val Ile Ala
            180                 185                 190
cct gcc acg gtg tcc gga ctc gtt gac gcc ggt cac gcc gtc aac gtg      624
Pro Ala Thr Val Ser Gly Leu Val Asp Ala Gly His Ala Val Asn Val
        195                 200                 205
gcg gca ttg ccg tat tcc atg agc tac gcg gcg ctc gaa gga ttc gaa      672
Ala Ala Leu Pro Tyr Ser Met Ser Tyr Ala Ala Leu Glu Gly Phe Glu
    210                 215                 220
gcc cac ggc gtc gac gcg gat tgg cgg ctg aag tac cgc agt gat tcc      720
Ala His Gly Val Asp Ala Asp Trp Arg Leu Lys Tyr Arg Ser Asp Ser
225                 230                 235                 240
tcc gac tgg aag ttc cag atg ctg cct ggc cgg ccc gac acc aat ctc      768
Ser Asp Trp Lys Phe Gln Met Leu Pro Gly Arg Pro Asp Thr Asn Leu
                245                 250                 255
tgg atc atg aat ccg agc aat ccg gac tgg cga gac cat att tcg gcc      816
Trp Ile Met Asn Pro Ser Asn Pro Asp Trp Arg Asp His Ile Ser Ala
            260                 265                 270
gag tat cag gat cag atc gac acg ttc ggg ttc gac ggc acc cac ctc      864
Glu Tyr Gln Asp Gln Ile Asp Thr Phe Gly Phe Asp Gly Thr His Leu
        275                 280                 285
gat cag ctc ggc aac tgg ggc agc ggg gca tcc gac ggt ggg atg aac      912
Asp Gln Leu Gly Asn Trp Gly Ser Gly Ala Ser Asp Gly Gly Met Asn
    290                 295                 300

gac gtg gcg ggg aat cct gtc gat att ccg gtc ggg ttc cgc gac ctg 960
Asp Val Ala Gly Asn Pro Val Asp Ile Pro Val Gly Phe Arg Asp Leu
305                 310                 315                 320
gtc gcc gaa acc aag gcc gcc acg ggg aag ccc gtc ggc ttc aac gcg 1008
Val Ala Glu Thr Lys Ala Ala Thr Gly Lys Pro Val Gly Phe Asn Ala
                325                 330                 335
gtc gac ggt ttc gcc ggc tcc acc ctg gcg agc tcg agc agt gac tac 1056
Val Asp Gly Phe Ala Gly Ser Thr Leu Ala Ser Ser Ser Ser Asp Tyr
            340                 345                 350
ctc tac acg gaa ctg tgg gag aac cac gaa acc tac tcg gag gtg cag 1104
Leu Tyr Thr Glu Leu Trp Glu Asn His Glu Thr Tyr Ser Glu Val Gln
        355                 360                 365
agc tac ctg gcc ggc cag cgg gcg gaa tcg gga ggc aag gcg gca gta 1152
Ser Tyr Leu Ala Gly Gln Arg Ala Glu Ser Gly Gly Lys Ala Ala Val
    370                 375                 380
acg gcc gcg tac ctg aac tac cgg agc aac acg ggt gat cgg tac gag 1200
Thr Ala Ala Tyr Leu Asn Tyr Arg Ser Asn Thr Gly Asp Arg Tyr Glu
385                 390                 395                 400
gcg gaa gac gga acc ctc atc ggg gtc cag gtc aac tcc aac cac ccc 1248
Ala Glu Asp Gly Thr Leu Ile Gly Val Gln Val Asn Ser Asn His Pro
                405                 410                 415
ggg tat acg ggt acc ggt ttc gtc gat cag ttc gga gac gtc ggc gac 1296
Gly Tyr Thr Gly Thr Gly Phe Val Asp Gln Phe Gly Asp Val Gly Asp
            420                 425                 430
agt gtc acg ctg agc gtc acg gtg ccg gag tca cgg cgc tac ggc atc 1344
Ser Val Thr Leu Ser Val Thr Val Pro Glu Ser Arg Arg Tyr Gly Ile
        435                 440                 445
gtc ccc gcc tgg gcc aac gac acg gcg aca acg gcc acc cgc acc gtg 1392
Val Pro Ala Trp Ala Asn Asp Thr Ala Thr Thr Ala Thr Arg Thr Val
    450                 455                 460
cga gtg gac gga gtg ccg gcc gga aag ctg aag atg acg ccg acg ggc 1440
Arg Val Asp Gly Val Pro Ala Gly Lys Leu Lys Met Thr Pro Thr Gly
465                 470                 475                 480
agc tgg gac tcc tgg aac acg gaa gcc ggc ttc ggc act tat ctg tcg 1488
Ser Trp Asp Ser Trp Asn Thr Glu Ala Gly Phe Gly Thr Tyr Leu Ser
                485                 490                 495
gcg ggg agc cac acg atc gag gtg tcc ctg gac gcc gga gat acc ggt 1536
Ala Gly Ser His Thr Ile Glu Val Ser Leu Asp Ala Gly Asp Thr Gly
            500                 505                 510
ttc gcg aac ctc gac agc atc acg ctc ggc acg ttc aac acg cca tcc 1584
Phe Ala Asn Leu Asp Ser Ile Thr Leu Gly Thr Phe Asn Thr Pro Ser
        515                 520                 525
gtt cag ttg gcg aat gct gcg ctc gcg gcg agt ggg gcc agc cat atc 1632
Val Gln Leu Ala Asn Ala Ala Leu Ala Ala Ser Gly Ala Ser His Ile
    530                 535                 540
gag atg ggc cag ggt gat caa atg ctc gtc gcg ccg tac ttc ctg gat 1680
Glu Met Gly Gln Gly Asp Gln Met Leu Val Ala Pro Tyr Phe Leu Asp
545                 550                 555                 560
gat tcc aag cag atg agc acc gaa ctg cag gcg tgg atg gag tcg tac 1728
Asp Ser Lys Gln Met Ser Thr Glu Leu Gln Ala Trp Met Glu Ser Tyr
                565                 570                 575
tac gac gtc atc acg ggc tac gag aac ctg ctg tac ggc ccg act ctc 1776
Tyr Asp Val Ile Thr Gly Tyr Glu Asn Leu Leu Tyr Gly Pro Thr Leu
            580                 585                 590
cgg caa ctg ccc aac gct gtt tcg atc gcg ggg cac acc acg agc acg 1824
Arg Gln Leu Pro Asn Ala Val Ser Ile Ala Gly His Thr Thr Ser Thr
        595                 600                 605
gac ggc acc ggc gac acc atc tgg gcg acg ccc atg cgc aac gac ggc 1872
Asp Gly Thr Gly Asp Thr Ile Trp Ala Thr Pro Met Arg Asn Asp Gly
    610                 615                 620
atg gac gtg ctg cac ctg atc aac ctg gag ggc aac gac ggt cgc tgg 1920
Met Asp Val Leu His Leu Ile Asn Leu Glu Gly Asn Asp Gly Arg Trp
625                 630                 635                 640

cga gaa ccg gcg gca cag gct ccg acg ttg tcg aat ctg ccg gtg aag 1968
Arg Glu Pro Ala Ala Gln Ala Pro Thr Leu Ser Asn Leu Pro Val Lys
                645                 650                 655
tac tac ctg gcc gac cgg ccg gtc ccc gcc gac gtc agg atc gcg agt 2016
Tyr Tyr Leu Ala Asp Arg Pro Val Pro Ala Asp Val Arg Ile Ala Ser
            660                 665                 670
ccg gac aac ggc ggc cga tcg agc acg ctg gca ttc aca tcc gga agc 2064
Pro Asp Asn Gly Gly Arg Ser Ser Thr Leu Ala Phe Thr Ser Gly Ser
        675                 680                 685
gac gcg ggc ggg aac tac atc tcc ttc acg gtg ccg gag ctg aag aac 2112
Asp Ala Gly Gly Asn Tyr Ile Ser Phe Thr Val Pro Glu Leu Lys Asn
    690                 695                 700
tgg agc ttc gtc tac ctc gga gac tcc acg agg gga aac ggc aac gtc 2160
Trp Ser Phe Val Tyr Leu Gly Asp Ser Thr Arg Gly Asn Gly Asn Val
705                 710                 715                 720
gtc acc cag gcg agc acg tgc ctc gac gtt gcg ggc ggc aac agc gcc 2208
Val Thr Gln Ala Ser Thr Cys Leu Asp Val Ala Gly Gly Asn Ser Ala
                725                 730                 735
gac gga acg gcc gtt cag gtc tgg aac tgt gcg agc gtt ccg gcc atg 2256
Asp Gly Thr Ala Val Gln Val Trp Asn Cys Ala Ser Val Pro Ala Met
            740                 745                 750
cag tgg acg ttc cag aac aac acg ctg tcg gcg ctc ggc aaa tgc ctg 2304
Gln Trp Thr Phe Gln Asn Asn Thr Leu Ser Ala Leu Gly Lys Cys Leu
        755                 760                 765
gat ctc gtc gcg ggc gga acc gcg gac ggc aca ctc gct cac ctc tgg 2352
Asp Leu Val Ala Gly Gly Thr Ala Asp Gly Thr Leu Ala His Leu Trp
    770                 775                 780
acg tgc tcc gac gtg ccc agt cag acc tgg gag cgt acc ggc cag ggg 2400
Thr Cys Ser Asp Val Pro Ser Gln Thr Trp Glu Arg Thr Gly Gln Gly
785                 790                 795                 800
cag tac ctc aat ccg gcc agc ggc aag tgc ctc gac ctg gtg ggt ggg 2448
Gln Tyr Leu Asn Pro Ala Ser Gly Lys Cys Leu Asp Leu Val Gly Gly
                805                 810                 815
tcg acc gcc aac gga acc aga gct cac ctg tgg agt tgc cac gcg ggt 2496
Ser Thr Ala Asn Gly Thr Arg Ala His Leu Trp Ser Cys His Ala Gly
            820                 825                 830
gcg agc cag aag tgg acg ctg ccc cag 2523
Ala Ser Gln Lys Trp Thr Leu Pro Gln
        835                 840

<210> 10
<211> 2529
<212> DNA
<213> Microbacterium trichothecenolyticum

<400> 10
atg cgt tct tcc ccc ttc aga cgc cga ctc acg gcg gtc aca atc gcg 48
Met Arg Ser Ser Pro Phe Arg Arg Arg Leu Thr Ala Val Thr Ile Ala
1               5                   10                  15
gga ctc gcc gtc gca gga atc atc gtt ccc gcg gct ccc gcc gca gcc 96
Gly Leu Ala Val Ala Gly Ile Ile Val Pro Ala Ala Pro Ala Ala Ala
            20                  25                  30
gcc gac ctc ggc gac gcg tgg acc gac aag gca cgc tac gca ccc ggc 144
Ala Asp Leu Gly Asp Ala Trp Thr Asp Lys Ala Arg Tyr Ala Pro Gly
        35                  40                  45
gag cag gtc acc gtc acc gcc gaa gta tcc ggc acc ggg tcc gtc cag 192
Glu Gln Val Thr Val Thr Ala Glu Val Ser Gly Thr Gly Ser Val Gln
    50                  55                  60
ttc tcc ctg gtg cac ctg gga gac gtg gtg gac acc gga aca gtc acc 240
Phe Ser Leu Val His Leu Gly Asp Val Val Asp Thr Gly Thr Val Thr
65                  70                  75                  80
gcc acc ggc tcg ggg cag gtc aac tgg acg gtg acg ccg ccc gcg aac 288
Ala Thr Gly Ser Gly Gln Val Asn Trp Thr Val Thr Pro Pro Ala Asn

                85                  90                  95
gac ttc acc ggt tac atg gtg cac gtc gat gcc ggg tcc agc acg acg 336
Asp Phe Thr Gly Tyr Met Val His Val Asp Ala Gly Ser Ser Thr Thr
            100                 105                 110
cag acc gct gtc gac gtg tcc agc aac tgg acg cga ttc cct cgc acc 384
Gln Thr Ala Val Asp Val Ser Ser Asn Trp Thr Arg Phe Pro Arg Thr
        115                 120                 125
ggg ttc ctc gac acc tac ccg atc gac ctg gac agc act gaa cag gcc 432
Gly Phe Leu Asp Thr Tyr Pro Ile Asp Leu Asp Ser Thr Glu Gln Ala
    130                 135                 140
gcc acg gta gac gaa ctg tcc cgc aat tac cac ctg aac tcg cta cag 480
Ala Thr Val Asp Glu Leu Ser Arg Asn Tyr His Leu Asn Ser Leu Gln
145                 150                 155                 160
ttc tac gac tgg atg tgg cgg cac gag aag cct gtg cag cga gag tcg 528
Phe Tyr Asp Trp Met Trp Arg His Glu Lys Pro Val Gln Arg Glu Ser
                165                 170                 175
aac ggg gac ctc gtc gac acc tgg acg gca tgg aac ggc gac gtc atc 576
Asn Gly Asp Leu Val Asp Thr Trp Thr Ala Trp Asn Gly Asp Val Ile
            180                 185                 190
gct ccg gac acc gtg gcc gga ctc atc gac gcc acc cat gac gtg aac 624
Ala Pro Asp Thr Val Ala Gly Leu Ile Asp Ala Thr His Asp Val Asn
        195                 200                 205
gcg gcc gcg ctg ccg tac tcc atg agc tac gca gcg ctg gag ggc ttc 672
Ala Ala Ala Leu Pro Tyr Ser Met Ser Tyr Ala Ala Leu Glu Gly Phe
    210                 215                 220
cag gcg cac ggg gtc gac gcc gac tgg cgg ctc aag tac cgc agc agc 720
Gln Ala His Gly Val Asp Ala Asp Trp Arg Leu Lys Tyr Arg Ser Ser
225                 230                 235                 240
ggg gat gac tgg aaa ttc cag atg ctt ccc aac gag ccc gac acc acc 768
Gly Asp Asp Trp Lys Phe Gln Met Leu Pro Asn Glu Pro Asp Thr Thr
                245                 250                 255
ctg tgg atc atg aac ccc gag aac ccc gac tgg gtc tcc cac atc acc 816
Leu Trp Ile Met Asn Pro Glu Asn Pro Asp Trp Val Ser His Ile Thr
            260                 265                 270
gaa cag tac gcc gac cag atc gac gcg ctc ggg ttc gac ggc acc cac 864
Glu Gln Tyr Ala Asp Gln Ile Asp Ala Leu Gly Phe Asp Gly Thr His
        275                 280                 285
atc gac caa ctc ggg aac tgg ggc gag gca gtg gac ggc ggc atg gac 912
Ile Asp Gln Leu Gly Asn Trp Gly Glu Ala Val Asp Gly Gly Met Asp
    290                 295                 300
gac gtc gcc ggt gac ccc gtc gac atc ccc cag ggc ttc gcg aac ctc 960
Asp Val Ala Gly Asp Pro Val Asp Ile Pro Gln Gly Phe Ala Asn Leu
305                 310                 315                 320
gtc gcg gcg acc aag gac gcc acc ggc aaa acc acc ggg ttc aac gcc 1008
Val Ala Ala Thr Lys Asp Ala Thr Gly Lys Thr Thr Gly Phe Asn Ala
                325                 330                 335
gtc gac gga ttc ggc agc gac tgg ctg gcc ggg tcc gag agc gac tac 1056
Val Asp Gly Phe Gly Ser Asp Trp Leu Ala Gly Ser Glu Ser Asp Tyr
            340                 345                 350
ctc tac acc gag ctg tgg gaa aac cac gaa acc tac gcg cag gtg cag 1104
Leu Tyr Thr Glu Leu Trp Glu Asn His Glu Thr Tyr Ala Gln Val Gln
        355                 360                 365
gac tac ctc gcc cag cag cgc gca gca tcc ggc ggc aag ggc gcc gtg 1152
Asp Tyr Leu Ala Gln Gln Arg Ala Ala Ser Gly Gly Lys Gly Ala Val
    370                 375                 380
ctc gcg gcc tac ctg aac tac cgc agc aac acc ggc gac cag tac gaa 1200
Leu Ala Ala Tyr Leu Asn Tyr Arg Ser Asn Thr Gly Asp Gln Tyr Glu
385                 390                 395                 400
gcc gaa gcc gga gtg ctc gcc ggc ggc gtc caa gtg gac tcc gac cac 1248
Ala Glu Ala Gly Val Leu Ala Gly Gly Val Gln Val Asp Ser Asp His
                405                 410                 415
acc ggg tat acc ggt acc ggg ttc atc gac tcc tac ggg gcc agc ggg 1296
Thr Gly Tyr Thr Gly Thr Gly Phe Ile Asp Ser Tyr Gly Ala Ser Gly

            420                 425                 430
gat gcc gtc acc gtg acg gta gcc gtg ccc gag agc cgc cgc tac gga 1344
Asp Ala Val Thr Val Thr Val Ala Val Pro Glu Ser Arg Arg Tyr Gly
        435                 440                 445
atc gtc ccc cgg tgg acg aat gga acc ggt gcg acg gcg acc cga acc 1392
Ile Val Pro Arg Trp Thr Asn Gly Thr Gly Ala Thr Ala Thr Arg Thr
    450                 455                 460
gtc acc gtg gac ggc acc aac gtg ggg cgc atc aag ctg cct tcc acg 1440
Val Thr Val Asp Gly Thr Asn Val Gly Arg Ile Lys Leu Pro Ser Thr
465                 470                 475                 480
gcc gac tgg gac acc tgg aac atc gaa ggt ggc acg gcc gcg tac ctg 1488
Ala Asp Trp Asp Thr Trp Asn Ile Glu Gly Gly Thr Ala Ala Tyr Leu
                485                 490                 495
acg gcc gga acc cac acc atc acc ctc gcc gtg ggc tcc ggg gac acc 1536
Thr Ala Gly Thr His Thr Ile Thr Leu Ala Val Gly Ser Gly Asp Thr
            500                 505                 510
ggc tac gtc aac ctc gac agc gtc acg ctg ggc act ttc gac aca ccc 1584
Gly Tyr Val Asn Leu Asp Ser Val Thr Leu Gly Thr Phe Asp Thr Pro
        515                 520                 525
tca gta cag ctc gcc aac gcc gcg ttc gcg gcg aac ggg gca tcg cac 1632
Ser Val Gln Leu Ala Asn Ala Ala Phe Ala Ala Asn Gly Ala Ser His
    530                 535                 540
atc gag atg ggc cag ggc gac cag atg ctc gtc gcg ccc tac ttc ctc 1680
Ile Glu Met Gly Gln Gly Asp Gln Met Leu Val Ala Pro Tyr Phe Leu
545                 550                 555                 560
gac gaa acc aag cag atg aac cac gaa ctg caa ggg tgg ttg gag aag 1728
Asp Glu Thr Lys Gln Met Asn His Glu Leu Gln Gly Trp Leu Glu Lys
                565                 570                 575
tac tac gac gtc atc acc ggc tac gag aac ctc ttc tac ggc ccc acc 1776
Tyr Tyr Asp Val Ile Thr Gly Tyr Glu Asn Leu Phe Tyr Gly Pro Thr
            580                 585                 590
ctc cgt cag cta tcg aac gcc gtg acc atc agc ggt caa ccg aca agc 1824
Leu Arg Gln Leu Ser Asn Ala Val Thr Ile Ser Gly Gln Pro Thr Ser
        595                 600                 605
acg gac ggc gct gcg aac acg gtc tgg acc aat gtg atg cgc aac gac 1872
Thr Asp Gly Ala Ala Asn Thr Val Trp Thr Asn Val Met Arg Asn Asp
    610                 615                 620
ggc atc gac gtc atc cat ctc atc aac ttg aag ggc aac aac ggc agc 1920
Gly Ile Asp Val Ile His Leu Ile Asn Leu Lys Gly Asn Asn Gly Ser
625                 630                 635                 640
tgg cgc gac ccg gcc gcg aca cca tcc acc ctc acc aac ctg ccc gtc 1968
Trp Arg Asp Pro Ala Ala Thr Pro Ser Thr Leu Thr Asn Leu Pro Val
                645                 650                 655
aag tac tac ctc ggc gat agc cca gtg cct gca tcc gtg cac gtt gcg 2016
Lys Tyr Tyr Leu Gly Asp Ser Pro Val Pro Ala Ser Val His Val Ala
            660                 665                 670
agc ccc gac cga gac ggc ggc cgt tcc acc gca ctg ccc ttc acc acg 2064
Ser Pro Asp Arg Asp Gly Gly Arg Ser Thr Ala Leu Pro Phe Thr Thr
        675                 680                 685
gga tcg gac gcg aac ggc acg tac ctg agc ttc acc gtg ccc gag ctg 2112
Gly Ser Asp Ala Asn Gly Thr Tyr Leu Ser Phe Thr Val Pro Glu Leu
    690                 695                 700
aag agc tgg gac ttc gtc tac ctg ggc gat tcc acc gcc ggc aac ggc 2160
Lys Ser Trp Asp Phe Val Tyr Leu Gly Asp Ser Thr Ala Gly Asn Gly
705                 710                 715                 720
aac gtc gtc acc aag gcg agc aag tgc ctc gac gtc gcc ggc gga agt 2208
Asn Val Val Thr Lys Ala Ser Lys Cys Leu Asp Val Ala Gly Gly Ser
                725                 730                 735
gcg gcg aac gga acc gct gtc cag atc tgg gac tgc gcc agc gtg ccc 2256
Ala Ala Asn Gly Thr Ala Val Gln Ile Trp Asp Cys Ala Ser Val Pro
            740                 745                 750
gcc cag gac tgg gac tac gcg ggc gag aag ctc tcc gcg ctg ggc aag 2304
Ala Gln Asp Trp Asp Tyr Ala Gly Glu Lys Leu Ser Ala Leu Gly Lys

        755                 760                 765
tgc ctc gac ctg tat cag ggt ggc acc gcg aac ggg acc ctc gcg cac        2352
Cys Leu Asp Leu Tyr Gln Gly Gly Thr Ala Asn Gly Thr Leu Ala His
    770                 775                 780
ctg tgg gac tgc gcc aac gtg ccc agc cag cag tgg gta cgg acc gcc        2400
Leu Trp Asp Cys Ala Asn Val Pro Ser Gln Gln Trp Val Arg Thr Ala
785                 790                 795                 800
cag tcg cag tac tac aac ccc gcc agc ggc cgg tgc ctc gat acc gtc        2448
Gln Ser Gln Tyr Tyr Asn Pro Ala Ser Gly Arg Cys Leu Asp Thr Val
                805                 810                 815
gga ggt ggt gcg gcg aac ggc acg cgc atc cac ctt tgg gat tgc cac        2496
Gly Gly Gly Ala Ala Asn Gly Thr Arg Ile His Leu Trp Asp Cys His
            820                 825                 830
gcc gga ccc agc cag aaa tgg acg ctg ccg ggc                            2529
Ala Gly Pro Ser Gln Lys Trp Thr Leu Pro Gly
        835                 840

<210>  11
<211>  36
<212>  DNA
<213>  Artificial Sequence

<400>  11
GGAGATATAC ATATGGCAAC CGTCGGCGAC GCGTGG                                  36

<210>  12
<211>  42
<212>  DNA
<213>  Artificial Sequence

<400>  12
GCTAGCCATA CCATGCTATT ACTGGGGCAG CGTCCACTTC TG                           42